# EUROPÄISCHE PATENTSCHRIFT

(11) **EP 0 861 241 B1**
(45) Veröffentlichungstag und Bekanntmachung des Hinweises auf die Patenterteilung: **14.04.2004**
(21) Anmeldenummer: 96938067.4
(22) Anmeldetag: 04.11.1996
(51) Int. Cl.: C07D 275/03, A01N 43/80, C07C 233/91

(54) **ACYLIERTE 5-AMINOISOTHIAZOLE MIT INSEKTIZIDER WIRKUNG, ZWISCHENPRODUKTE UND VERFAHREN ZU IHRER HERSTELLUNG**
ACYLATED 5-AMINOISOTHIAZOLES WITH INSECTICIDAL PROPERTIES, INTERMEDIATE PRODUCTS AND PROCESS FOR PRODUCING THEM
5-AMINOISOTHIAZOLES ACYLES AVEC EFFET INSECTICIDE, PRODUITS INTERMEDIAIRES ET PROCEDE DE PRODUCTION CORRESPONDANT

(30) Priorität: 14.11.1995 DE 19542372
(43) Veröffentlichungstag der Anmeldung: 02.09.1998
(73) Patentinhaber: Bayer CropScience AG, 40789 Monheim (DE)
(72) Erfinder: HEIL, Markus, D-51381 Leverkusen (DE); ERDELEN, Christoph, D-42799 Leichlingen (DE); WACHENDORFF-NEUMANN, Ulrike, D-56566 Neuwied (DE); TURBERG, Andreas, D-40699 Erkrath (DE); MENCKE, Norbert, D-51381 Leverkusen (DE)
(86) Internationale Anmeldenummer: PCT/EP1996/004796
(87) Internationale Veröffentlichungsnummer: WO 1997/018198

(56) Entgegenhaltungen:
- EP-A- 0 334 809
- EP-A- 0 623 282
- EP-A- 0 640 597
- WO-A-95/31448
- FR-A- 2 014 527
- CHEM.BER., Bd. 96, Februar 1963, WEINHEIM, Seiten 526-533, XP000651464 GOERDELER,J. ET AL.: "Darstellung und Cyclisierung von alpha-Acyl-beta- amino-thiocrotonamiden"
- CHEM.BER., Bd. 97, Oktober 1964, WEINHEIM, Seiten 3106-3117, XP000651463 GOERDELER,J. ET AL.: "Synthese von 5-Amino-3-hydroxy(alkoxy-,amino-)isothiazo len und von Derivaten der Pyrimidinthione-(4) "
- INDIAN J.CHEM., Bd. 16B, September 1978, Seiten 752-754, XP002026306 RAJAPPA,S. ET AL.: "Synthesis of Thiophenes : Part VI - Synthesis of 2-Nitro- & 2,4-Dinitrothiophenes by direct Ring-closure Reactions"
- J.ORG.CHEM., Bd. 42, Nr. 20, 1977, WASHINGTON, Seiten 3230-3233, XP000651467 HOWE,R.K.: "Reaction of Ethyl-beta-Aminocrotonate with Trichloromethanesulfenyl Chloride"

## Beschreibung

Die vorliegende Erfindung betrifft neue acylierte 5-Aminoisothiazole, Verfahren zu ihrer Herstellung und ihre Verwendung zur Bekämpfung von tierischen Schädlingen.

Es ist bereits bekannt, daß bestimmte acylierte 4-Cyano-5-aminoisothiazole insektizide Eigenschaften aufweisen (vgl. z.B. EP-A 0 623 282).

Die Wirksamkeit und Wirkungsbreite dieser Verbindungen ist jedoch insbesondere bei niedrigen Aufwandmengen und Konzentrationen nicht immer voll zufriedenstellend.

Es wurden neue acylierte 5-Aminoisothiazole der Formeln (IA) und (IB) gefunden, 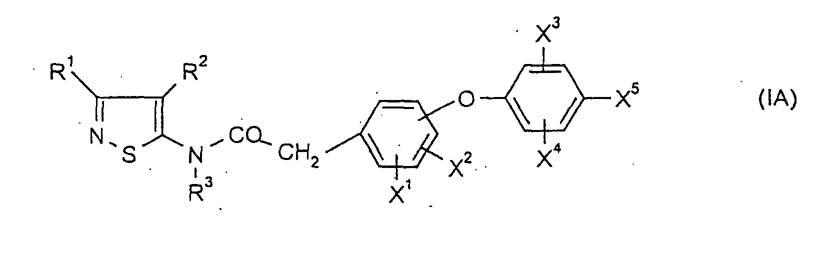 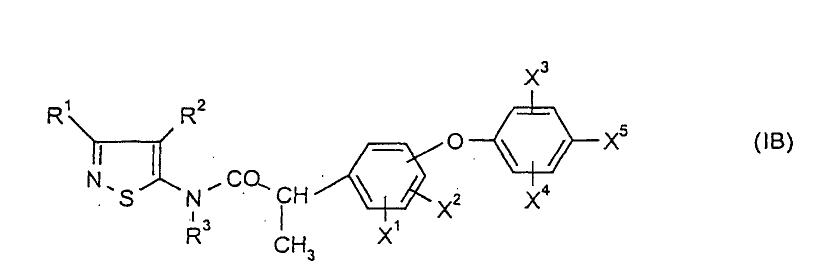 in welchen
- R¹: für C₁-C₄-Alkyl, C₁-C₄-Halogenalkyl mit 1 bis 5 gleichen oder verschiedenen Halogenatomen, wie Fluor-, Chlor- und Bromatomen, C₁-C₄-Alkoxy-C₁-C₄-alkyl, C₁-C₄-Alkylthio-C₁-C₄-alkyl, C₁-C₄-Alkoxy, C₁-C₄-Alkylthio oder für gegebenenfalls einfach bis dreifach, gleich oder verschieden durch C₁-C₄-Alkyl oder Halogen substituiertes C₃-C₆-Cycloalkyl steht,
- R²: für Wasserstoff, Halogen, Cyano, Nitro, Thiocyanato, C₁-C₄-Alkoxycarbonyl, C₂-C₄-Alkenyloxy-carbonyl, C₁-C₄-Alkylthio, C₁-C₄-Halogenalkylthio mit 1 bis 5 gleichen oder verschiedenen Halogenatomen, wie Fluor- und Chlor, C₁-C₄-Alkylsulfinyl, C₁-C₄-Halogenalkylsulfinyl mit 1 bis 5 gleichen oder verschiedenen Halogenatomen, wie Fluor- und Chloratomen, C₁-C₄-Alkylsulfonyl, C₁-C₄-Halogenalkylsulfonyl mit 1 bis 5 gleichen oder verschiedenen Halogenatomen, wie Fluor- und Chloratomen oder für Thiocarbamoyl steht,
- R³: für Wasserstoff, C₁-C₄-Alkyl, C₁-C₄-Halogenalkyl mit 1 bis 5 gleichen oder verschiedenen Halogenatomen, wie Fluor- und Chloratomen, C₁-C₄-Alkoxy-C₁-C₄-alkyl, C₁-C₄-Alkyl-carbonyl, C₁-C₄-Alkylsulfonyl, jeweils gegebenenfalls einfach bis dreifach, gleich oder verschieden im Phenylring substituiertes Phenylcarbonyl, Phenylsulfonyl oder Benzyl, wobei als Substituenten jeweils Halogen, Nitro, Cyano, C₁-C₄-Alkyl, C₁-C₂-Halogenalkyl mit 1 bis 5 gleichen oder verschiedenen Halogenatomen, wie Fluor- und Chloratome, C₁-C₄-Alkoxy, C₁-C₄-Alkylthio, C₁-C₂-Halogenalkoxy mit 1 bis 5 gleichen oder verschiedenen Halogenatomen, wie Fluor- und Choratome oder C₁-C₂-Halogenalkylthio mit 1 bis 5 gleichen oder verschiedenen Halogenatomen, wie Fluor- und Chloratome in Frage kommen, oder für gegebenenfalls einfach bis dreifach, gleich oder verschieden durch C₁-C₄-Alkyl oder Halogen substituiertes C₃-C₆-Cycloalkyl steht,
- X¹, X², X³ und X⁴: für Wasserstoff, Halogen, Nitro, Cyano, C₁-C₁₂-Alkyl, C₁-C₁₂-Halogenalkyl mit 1 bis 5 gleichen oder verschiedenen Halogenatomen, wie Fluor- und Chloratome, C₁-C₁₂-Alkoxy, C₁-C₁₂-Halogenalkoxy mit 1 bis 5 gleichen oder verschiedenen Halogenatomen, wie Fluor- und Chloratome, C₁-C₁₂-Alkylthio, C₁-C₁₂-Halogenalkylthio mit 1 bis 5 gleichen oder verschiedenen Halogenatomen, wie Fluor- und Chloratomen, C₂-C₁₂-Alkenyl, C₂-C₁₂-Halogenalkenyl mit 1 bis 5 gleichen oder verschiedenen Halogenatomen, wie Fluor- und Chloratomen, gegebenenfalls einfach bis dreifach, gleich oder verschieden durch C₁-C₄-Alkyl oder Halogen substituiertes C₃-C₈-Cycloalkyl, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen Halogenatomen, wie Fluor- und Chloratomen, C₁-C₄-Alkyl-carbonyl, C₁-C₄-Alkoxy-carbonyl, Thioamid, C₁-C₄-Alkoximino-C₁-C₄-alkyl oder gegebenenfalls durch C₁-C₄-Alkyl substituiertes Oxadiazolyl stehen, C₁-C₄-Alkylthio, C₁-C₄-Halogenalkylthio mit 1 bis 5 gleichen oder verschiedenen Halogenatomen, wie Fluor- und Chloratome, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen Halogenatomen, wie Fluor- und Chloratomen, C₁-C₄-Alkoxy-carbonyl, Thioamid, C₁-C₄-Alkoximino-C₁-C₄-alkyl oder gegebenenfalls durch C₁-C₄-Alkyl substituiertes Oxdiazolyl stehen,
- X⁵: für Halogen, Nitro, Cyano, C₁-C₁₂-Alkyl, C₁-C₁₂-Halogenalkyl mit 1 bis 5 gleichen oder verschiedenen Halogenatomen, wie Fluor- und Chloratome, C₁-C₁₂-Alkoxy, C₁-C₁₂-Halogenalkoxy mit 1 bis 5 gleichen oder verschiedenen Halogenatomen, wie Fluor- und Chloratome, C₁-C₁₂-Alkylthio, C₁-C₁₂-Halogenalkylthio mit 1 bis 5 gleichen oder verschiedenen Halogenatomen, wie Fluor- und Chloratomen, C₂-C₁₂-Alkenyl, C₂-C₁₂-Halogenalkenyl mit 1 bis 5 gleichen oder verschiedenen Halogenatomen, wie Fluor- und Chloratomen, gegebenenfalls einfach bis dreifach, gleich oder verschieden durch C₁-C₄-Alkyl oder Halogen substituiertes C₃-C₈-Cycloalkyl, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen Halogenatomen, wie Fluor- und Chloratomen, C₁-C₄-Alkyl-carbonyl, C₁-C₄-Alkoxy-carbonyl, Thioamid, C₁-C₄-Alkoximino-C₁-C₄-alkyl oder gegebenenfalls durch C₁-C₄-Alkyl substituiertes Oxadiazolyl stehen, C₁-C₄-Alkylthio, C₁-C₄-Halogenalkylthio mit 1 bis 5 gleichen oder verschiedenen Halogenatomen, wie Fluor- und Chloratome, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen Halogenatomen, wie Fluor- und Chloratomen, C₁-C₄-Alkoxy-carbonyl, Thioamid, C₁-C₄-Alkoximino-C₁-C₄-alkyl oder gegebenenfalls durch C₁-C₄-Alkyl substituiertes Oxdiazolyl stehen.

Weiterhin wurde gefunden, daß man die acylierten 5-Aminoisothiazole der Formeln (IA) und (IB) erhält, wenn man
a) 5-Aminoisothiazole der Formel (II) 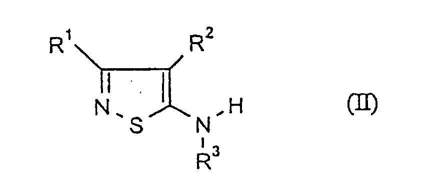 in welcher
   - R¹, R² und R³: die in Anspruch 1 angegebene Bedeutung haben,
   mit Säurehalogeniden der Formel (III)

   R⁴-Y-CO-Hal (III)

   in welcher
   - R⁴-Y: für den Rest 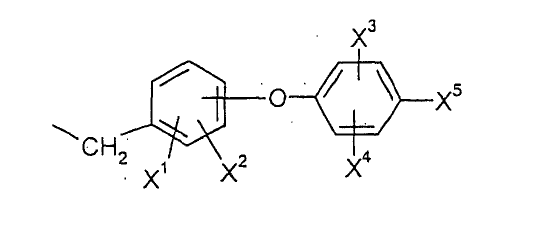 oder für den Rest 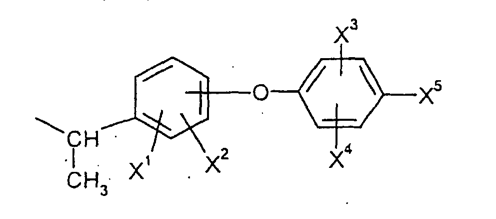 steht und X¹, X², X³, X⁴ und X⁵ die in einem der vorstehenden Ansprüche angegebenen Bedeutungen haben,
   - Hal: für Halogen steht,
   in Gegenwart einer Base und in Gegenwart eines Verdünnungsmittels umsetzt;
   oder
b) acylierte 5-Aminoisothiazole der Formel (Ia) 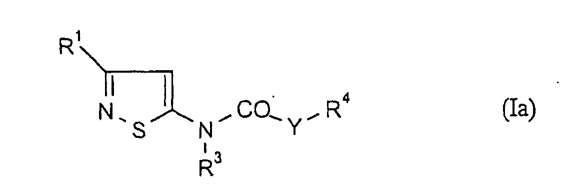 in welcher
   - R¹ und R³: die in den vorstehenden Ansprüchen angegebene Bedeutung haben, und
   - R⁴-Y-: die unter a) angegebene Bedeutung hat,
   (α) mit einem Halogenierungsmittel, gegebenenfalls in Gegenwart eines Verdünnungsmittels sowie gegebenenfalls in Gegenwart eines Katalysators umsetzt,
      oder
   (β) mit einem Nitrierungsreagenz, gegebenenfalls in Gegenwart eines Verdünnungsmittels umsetzt,
   oder
c) β-Amino-thiocrotonamide der Formel (IV) 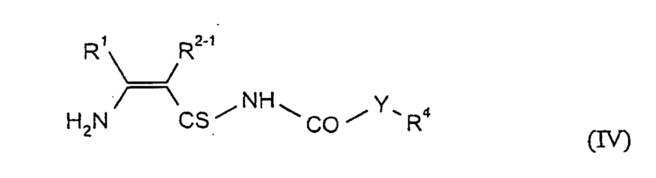 in welcher
   - R¹: die in den vorstehenden Ansprüchen angegebene Bedeutung hat,
   - R⁴-Y-: die unter a) angegebene Bedeutung hat und
   - R²⁻¹: für Cyano oder Alkoxycarbonyl steht,
   mit einem Oxidationsmittel, gegebenenfalls in Gegenwart eines Verdünnungsmittels cyclisiert.

Schließlich wurde gefunden, daß die neuen acylierten 5-Aminoisothiazole der Formeln (IA) und (IB) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die erfindungsgemäßen acylierten 5-Aminoisothiazole sind durch die Formeln (IA) und (IB) allgemein definiert.

Bevorzugte Substituenten bzw. Bereiche der in den oben und nachstehend erwähnten Formeln aufgeführten Reste werden im folgenden erläutert.
- R¹: steht bevorzugt für Methyl, Ethyl, n- oder i-Propyl, n-, i-, s- oder t-Butyl; CH₂F, CHF₂, CF₃, CH₂Cl, CH₂Br, CHClCH₃; Methoxy, Ethoxy, Methoxymethyl, Ethoxymethyl; Methylthiomethyl oder Cyclopropyl.
- R²: steht bevorzugt für Wasserstoff, Chlor, Brom, Cyano, Nitro, Thiocyanato; Methoxycarbonyl, Ethoxycarbonyl, i-Propoxycarbonyl, n-Propoxycarbonyl; Allyloxycarbonyl; SCF₃, SCCl₂F, SOCF₃, SOCCl₂F, SO₂CF₃, SO₂CCl₂F, SCHF₂, SOCHF₂, SO₂CHF₂ oder CSNH₂.
- R³: steht bevorzugt für Wasserstoff, Methyl, Ethyl, n- oder i-Propyl; Methoxymethyl, Ethoxymethyl, n-Propoxymethyl, n-Butoxymethyl; Methylcarbonyl, Methylsulfonyl; jeweils gegebenenfalls einfach oder zweifach, gleich oder verschieden durch Fluor, Chlor, Methyl oder Trifluormethyl substituiertes Phenylcarbonyl oder Benzyl; oder Cyclopropyl.
- X¹, X², X³ and X⁴: stehen bevorzugt für Wasserstoff Halogen, Nitro, Cyano, C₁-C₁₂-Alkyl, C₁-C₁₂-Halogenalkyl mit 1 bis 5 gleichen oder verschiedenen Halogenatomen, wie Fluor- und Chloratome, C₁-C₄-Alkoxy, C₁-C₄-Halogenalkoxy mit 1 bis 5 gleichen oder verschiedenen Halogenatomen, wie Fluor- und Choratome, C₁-C₄-Alkylthio, C₁-C₄-Halogenalkylthio mit 1 bis 5 gleichen oder verschiedenen Halogenatomen, wie Fluor - und Chloratome, C₂-C₄-Alkenyl, C₂-C₄-Halogenalkenyl mit 1 bis 5 gleichen oder verschiedenen Halogenatomen, wie Fluor- und Chloratome sowie jeweils gegebenenfalls einfach bis dreifach, gleich oder verschieden durch Halogen, Nitro, Cyano, C₁-C₄-Alkyl, C₁-C₄-Halogenalkyl mit 1 bis 5 gleichen oder verschiedenen Halogenatomen, wie Fluor- und Chloratome, C₁-C₄-Alkoxy, C₁-C₄-Halogenalkoxy mit 1 bis 5 gleichen oder verschiedenen Halogenatomen, wie Fluor- und Chloratome, C₁-C₄-Alkylthio, C₁-C₄-Halogenalkylthio mit 1 bis 5 gleichen oder verschiedenen Halogenatomen, wie Fluor- oder Chloratome, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen. Halogenatomen, wie Fluor- oder Chloratomen, C₁-C₄-Alkylcarbonyl, C₁-C₄-Alkoxy-carbonyl, Thioamid, C₁-C₄-Alkoximino-C₁-C₂-alkyl oder gegebenenfalls durch C₁-C₂-Alkyl substituiertes 1,2,4-Oxdiazol-3-yl.
- X⁵: steht bevorzugt für Halogen, Nitro, Cyano, C₁-C₁₂-Alkyl, C₁-C₁₂-Halogenalkyl mit 1 bis 5 gleichen oder verschiedenen Halogenatomen, wie Fluor- und Chloratome, C₁-C₄-Alkoxy, C₁-C₄-Halogenalkoxy mit 1 bis 5 gleichen oder verschiedenen Halogenatomen, wie Fluor- und Choratome, C₁-C₄-Alkylthio, C₁-C₄-Halogenalkylthio mit 1 bis 5 gleichen oder verschiedenen Halogenatomen, wie Fluor - und Chloratome, C₂-C₄-Alkenyl, C₂-C₄-Halogenalkenyl mit 1 bis 5 gleichen oder verschiedenen Halogenatomen, wie Fluor- und Chloratome sowie jeweils gegebenenfalls einfach bis dreifach, gleich oder verschieden durch Halogen, Nitro, Cyano, C₁-C₄-Alkyl, C₁-C₄-Halogenalkyl mit 1 bis 5 gleichen oder verschiedenen Halogenatomen, wie Fluor- und Chloratome, C₁-C₄-Alkoxy, C₁-C₄-Halogenalkoxy mit 1 bis 5 gleichen oder verschiedenen Halogenatomen, wie Fluor- und Chloratome, C₁-C₄-Alkylthio, C₁-C₄-Halogenalkylthio mit 1 bis 5 gleichen oder verschiedenen Halogenatomen, wie Fluor- oder Chloratome, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen Halogenatomen, wie Fluor- oder Chloratomen, C₁-C₄-Alkylcarbonyl, C₁-C₄-Alkoxy-carbonyl, Thioamid, C₁-C₄-Alkoximino-C₁-C₂-alkyl oder gegebenenfalls durch C₁-C₂-Alkyl substituiertes 1,2,4-Oxdiazol-3-yl.

- R¹: steht besonders bevorzugt für Methyl, Ethyl, n- oder i-Propyl, n-, i-, s- oder t-Butyl; CH₂F, CHF₂, CF₃, CH₂Cl, CH₂Br, Methoxy oder Ethoxy.
- R²: steht besonders bevorzugt für Wasserstoff, Chlor, Brom, Cyano, Nitro, Thiocyanato, Methoxycarbonyl, Ethoxycarbonyl, SCF₃, SOCF₃ oder SO₂CF₃.
- R³: steht besonders bevorzugt für Wasserstoff, Methyl, Ethyl, Methoxymethyl, Ethoxymethyl, Methylcarbonyl, Phenylcarbonyl oder Methylsulfonyl.
- X¹, X², X³ und X⁴: stehen besonders bevorzugt für Wasserstoff, Fluor, Chor, Brom, Nitro, Cyano, Methyl, Ethyl, n- oder i-Propyl, n-, i-, s- oder t-Butyl; Methoxy, Ethoxy, n- oder i-Propoxy, n-, i-, s- oder t-Butoxy; Methylthio, CF₃, OCF₃, OCHF₂, SCF₃, SCCl₂F; CH₂Br, CH₂Cl sowie gegebenenfalls einfach bis dreifach, gleich oder verschieden durch Fluor, Chlor, Brom, Nitro, Cyano, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Methylthiomethyl, CF₃, OCF₃, OCHF₂, SCF₃, SCCl₂F, CH₂Br, CH₂Cl, Acetyl, Ethylcarbonyl, Methoxycarbonyl, Methylsulfonyl, Trifluormethylsulfonyl, Thioamid, Methoximinomethyl, 1-(Methoximino)ethyl, 1-(Ethoximino)ethyl, 1,2,4-Oxdiazol-3-yl oder 5-Methyl-1,2,4-oxdiazol-3-yl.
- X⁵: steht besonders bevorzugt für Fluor, Chor, Brom, Nitro, Cyano, Methyl, Ethyl, n- oder i-Propyl, n-, i-, s - oder t-Butyl; Methoxy, Ethoxy, n- oder i-Propoxy, n-, i-, s- oder t-Butoxy; Methylthio, CF₃, OCF₃, OCHF₂, SCF₃, SCCl₂F; CH₂Br, CH₂Cl sowie gegebenenfalls einfach bis dreifach, gleich oder verschieden durch Fluor, Chlor, Brom, Nitro, Cyano, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Methylthiomethyl, CF₃, OCF₃, OCHF₂, SCF₃, SCCl₂F, CH₂Br, CH₂Cl, Acetyl, Ethylcarbonyl, Methoxycarbonyl, Methylsulfonyl, Trifluormethylsulfonyl, Thioamid, Methoximinomethyl, 1-(Methoximino)ethyl, 1-(Ethoximino)ethyl, 1,2,4-Oxdiazol-3-yl oder 5-Methyl-1,2,4-oxidazol-3-yl.
- X¹, X², X³ und X⁴: stehen ganz besonders bevorzugt für Wasserstoff.

Erfindungsgemäß ganz besonders bevorzugt werden die Verbindungen der Formeln (IA), (IB), in welchen eine Kombination der vorstehend als ganz besonders bevorzugt aufgeführten Bedeutungen vorliegt.

In den oben und nachstehend aufgeführten Restedefinitionen sind Kohlenwasserstoffreste, wie Alkyl oder Alkenyl - auch in Verbindung mit Heteroatomen wie Alkoxy oder Alkylthio - soweit möglich jeweils geradkettig oder verzweigt.

Im einzelnen seien neben den Herstellungsbeispielen die folgenden Verbindungen der Formel (IC) genannt:

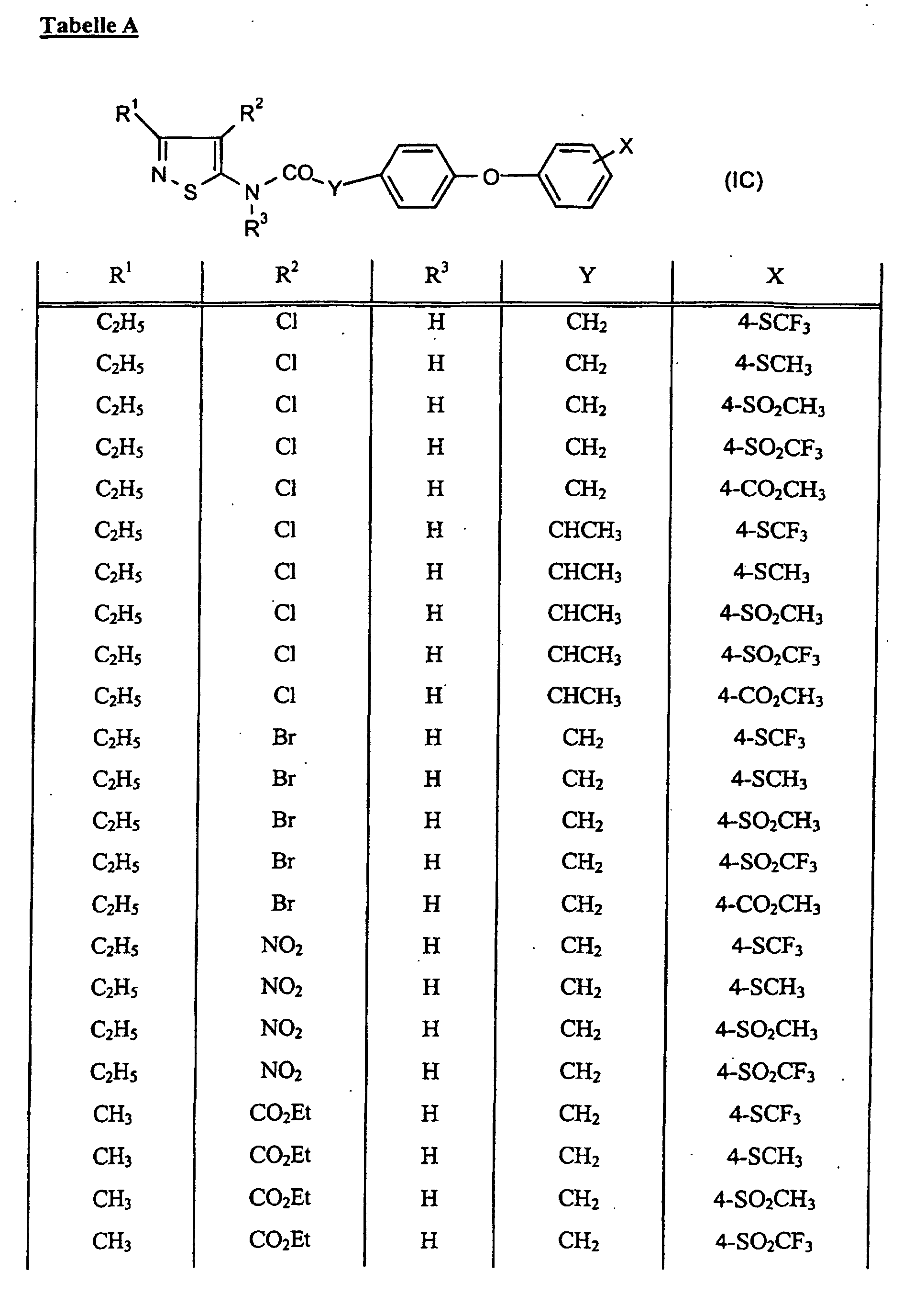

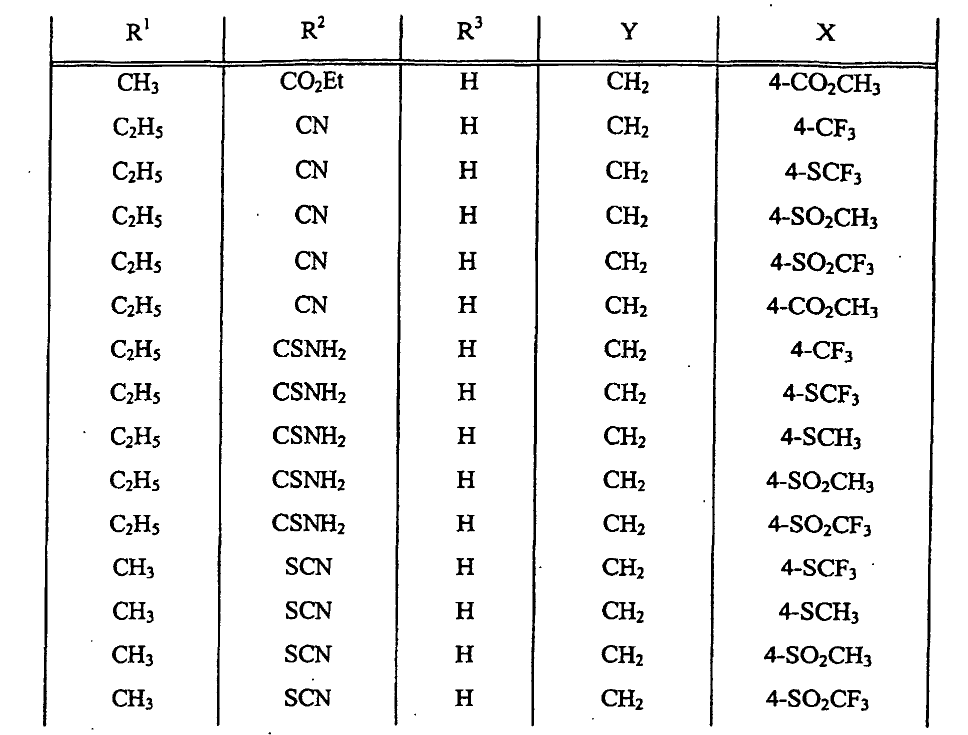

Verwendet man bei der Herstellung von Verbindungen der Formeln (IA), (IB) gemäß Verfahren (a) z.B. 5-Amino-3-methyl-isothiazol und [4-(4-Cyano)phenoxy]phenylessigsäurechlorid als Ausgangsstoffe, so kann der Reaktionsablauf durch das folgende Reaktionsschema wiedergegeben werden:

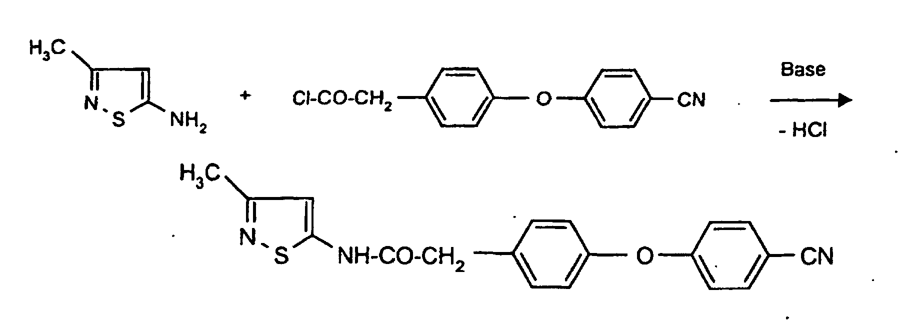

Verwendet man bei der Herstellung von Verbindungen der Formeln (IA), (IB) gemäß Verfahren (b/α) z.B. 5-[4-(4-Cyano)phenoxy]-phenylacetylamino-3-methyl-isothiazol und Brom als Ausgangsstoffe, so kann der Reaktionsablauf durch das folgende Reaktionsschema wiedergegeben werden:

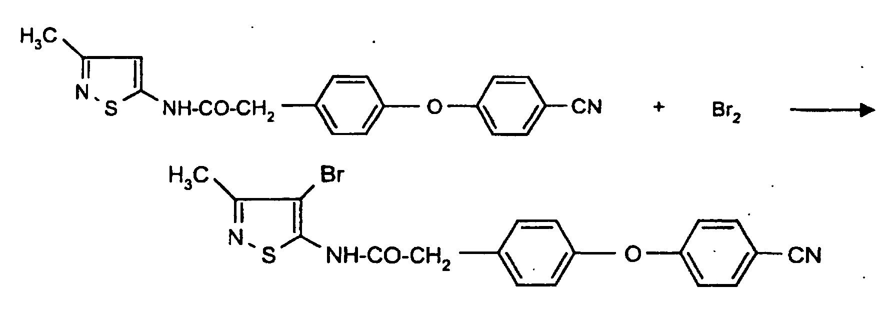

Verwendet man bei der Herstellung von Verbindungen der Formeln (IA), (IB) gemäß Verfahren (b/β) z.B. 5-[4-(4-Cyano)phenoxy]-phenylacetylamino-3-methyl-isothiazol und Salpetersäure, gegebenenfalls in Gegenwart von Essigsäure als Ausgangsstoffe, so kann der Reaktionsablauf durch das folgende Reaktionsschema wiedergegeben werden:

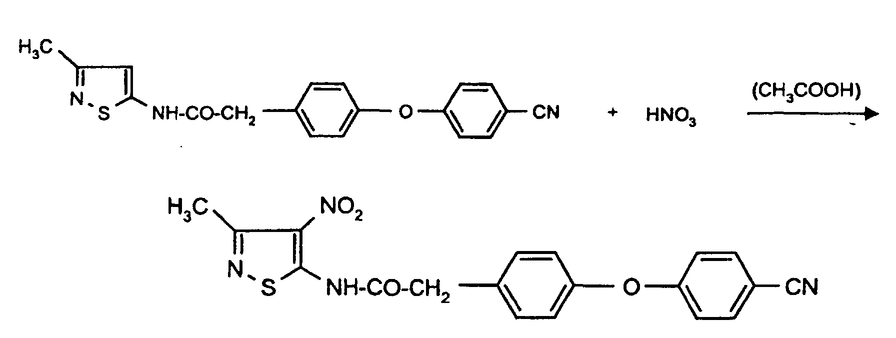

Verwendet man bei der Herstellung von Verbindungen der Formeln (IA), (IB) gemäß Verfahren (c) z.B. β-Amino-α-ethoxy-carbonyl-thiocrotonsäure-[4-(4-cyano)phenoxyphenylacetyl]amid als Ausgangsstoff und H₂O₂ als Oxidationsmittel, so kann der Reaktionsablauf durch das folgende Reaktionsschema wiedergegeben werden:

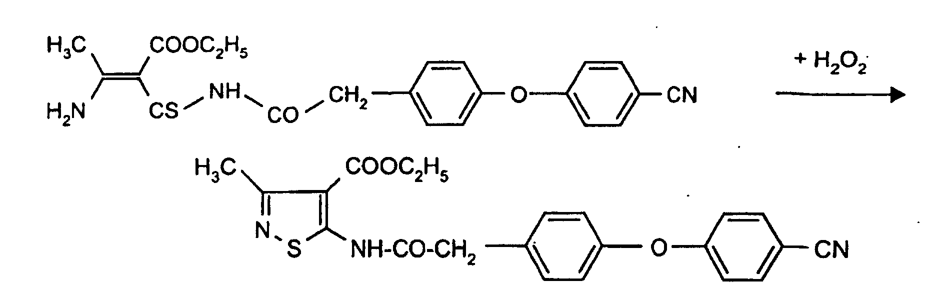

Die beim erfindungsgemäßen Verfahren (a) als Ausgangsstoffe zu verwendenden 5-Aminoisothiazole der Formel (II) sind bekannt (vgl. z.B. DE-A 4328425, DE-A 2249162, WO-A 93/19054, WO-A 94/21617 oder J. Het. Chem. 1989, 26, 1575) und/oder lassen sich nach bekannten Methoden herstellen (vgl. z.B. die o.g. Literaturstellen).

Die weiterhin beim erfindungsgemäßen Verfahren (a) als Ausgangsstoffe zu verwendenden Säurehalogenide der Formel (III) sind allgemein bekannte Verbindungen der Organischen Chemie. In der Formel (III) steht Hal vorzugsweise für Chlor oder Brom.

Die beim erfindungsgemäßen Verfahren (c) als Ausgangsstoffe zu verwendenden β-Amino-thiocrotonamide sind durch die Formel (IV) allgemein definiert. In dieser Formel stehen R¹, R⁴ und Y vorzugsweise, besonders bevorzugt sowie ganz besonders bevorzugt für diejenigen Bedeutungen, die bereits oben im Zusammenhang mit der Beschreibung der erfindungsgemäßen Verbindungen der Formeln (IA), (IB) als bevorzugt, besonders bevorzugt sowie ganz besonders bevorzugt für R¹, R⁴ und Y genannt wurden. R²⁻¹ steht bevorzugt für Cyano oder C₁-C₄-Alkoxycarbonyl, besonders bevorzugt für Cyano, Methoxycarbonyl, Ethoxycarbonyl, i-Propoxycarbonyl oder n-Propoxycarbonyl und ganz besonders bevorzugt für Cyano, Methoxycarbonyl oder Ethoxycarbonyl.

Die β-Amino-thiocrotonamide der Formel (IV) sind neu. Sie können jedoch in allgemein bekannter Art und Weise erhalten werden, indem man Säurehalogenide der Formel (III) mit Thiocyanaten der Formel (V)

M-SCN (V)

in welcher
- M: für ein Alkalimetall, vorzugsweise Kalium oder Natrium steht,
gegebenenfalls in Gegenwart eines Verdünnungsmittels, beispielsweise eines halogenierten aliphatischen oder aromatischen Kohlenwasserstoffs, eines Ethers, Nitrils oder Amids bei Temperaturen zwischen -40°C und +120°C, vorzugsweise zwischen 0°C bis 80°C umsetzt
und die dabei entstehenden Verbindungen der Formel (VI)

R⁴-Y-CO-NCS (VI)

in welcher
- R⁴ und Y: die oben angegebene Bedeutung haben,
vorzugsweise ohne Isolierung direkt mit Verbindungen der Formel (VII) 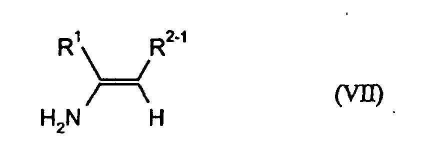 in welcher
- R¹ und R²⁻¹: die oben angegebene Bedeutung haben,
gegebenenfalls in Gegenwart eines Verdünnungsmittels, beispielsweise eines halogenierten aliphatischen oder aromatischen Kohlenwasserstoffs, eines Ethers, Nitrils oder Amids bei Temperaturen zwischen -40°C und 120°C, vorzugsweise zwischen 0°C und 80°C umsetzt (vgl. auch die allgemeinen Verfahrensangaben in J. Org. Chem. 1977, 20, 3230 und Chem. Ber. 1961, 94, 2950).

Die Thiocyanate der Formel (V) und die Verbindungen der Formel (VII) sind allgemein bekannte Verbindungen der Organischen Chemie.

Das oben beschriebene Verfahren (a) zur Herstellung der Verbindungen der Formeln (IA), (IB) wird in Gegenwart eines Verdünnungsmittels durchgeführt. Als Verdünnungsmittel können alle üblichen Lösungsmittel eingesetzt werden.

Vorzugsweise verwendbar sind gegebenenfalls halogenierte aliphatische oder aromatische Kohlenwasserstoffe, Ether oder Nitrile wie z.B. Cyclohexan, Toluol, Chlorbenzol, Choroform, Dichlormethan, Dichlorethan, Dioxan, Tetrahydrofuran, Diethylether oder Acetonitril.

Das oben beschriebene Verfahren (a) zur Herstellung der Verbindungen der Formeln (IA), (IB) wird in Gegenwart einer Base durchgeführt.

Als Basen können beim Verfahren (a) alle üblichen Protonenakzeptoren eingesetzt werden. Vorzugsweise verwendbar sind Alkali- oder Erdalkalihydroxide, Alkali- oder Erdalkalicarbonate oder -hydrogencarbonate oder Stickstoffbasen. Genannt seien beispielsweise Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Die Reaktionstemperaturen können bei dem beschriebenen Verfahren (a) in einem größeren Bereich variiert werden. Im Allgemeinen arbeitet man bei Temperaturen zwischen -40°C und +200°C, bevorzugt zwischen 0°C und 100°C.
Bei der Durchführung des oben beschriebenen Verfahrens (a) zur Herstellung der Verbindungen der Formeln (IA), (IB) setzt man im Allgemeinen pro Mol 5-Aminoisothiazol der Formel (II) 1 bis 2 Mol, vorzugsweise 1 bis 1,5 Mol an Säurehalogenid der Formel (III) ein.

Dabei erweist es sich in manchen Fällen als vorteilhaft, die 5-Aminoisothiazole der Formel (II) in Form ihrer Hydrohalogenide, wie insbesondere als Hydrochloride einzusetzen.

Aufarbeitung und Isolierung der Endprodukte erfolgen in allgemein bekannter Art und Weise.

Das oben beschriebene Verfahren (b/a) zur Herstellung der Verbindungen der Formeln (IA), (IB) wird mittels eines Halogenierungsmittels durchgeführt.

Hierfür können alle üblichen Halogenierungsmittel eingesetzt werden. Vorzugsweise verwendbar sind Cl₂, Br₂, Halogensauerstoffsäuren oder deren Salze, wie beispielsweise Natrium- oder Kaliumhypochlorit und -bromit, SO₂Cl₂, S₂Cl₂, PCl₅ oder N-Bromsuccinimid.

Das oben beschriebene Verfahren (b/α) zur Herstellung der Verbindungen der Formeln (IA), (IB) wird gegebenenfalls in Gegenwart eines Verdünnungsmittels durchgeführt. Vorzugsweise verwendbar sind gegebenenfalls halogenierte aliphatische oder aromatische Kohlenwasserstoffe, Ether, Nitrile oder Amide, wie z.B. Cyclohexan, Toluol, Chlorbenzol, Chloroform, Dichlormethan, Dichlorethan, Dioxan, Tetrahydrofuran, Diethylether, Acetonitril oder Dimethylformamid.

Das oben beschriebene Verfahren (b/α) zur Herstellung der Verbindungen der Formeln (IA), (IB) wird gegebenenfalls in Gegenwart eines Katalysators durchgeführt. Als Katalysatoren können alle für eine Halogenierung üblichen sauren oder basischen Katalysatoren eingesetzt werden, wie z.B. Halogenwasserstoffe oder Natriumacetat, ferner Radikalstarter wie Azoisobutyronitril oder Dibenzoylperoxid.

Die Reaktionstemperaturen können bei dem oben beschriebenen Verfahren (b/α) in einem größeren Bereich variiert werden. Im Allgemeinen arbeitet man bei Temperaturen zwischen -40°C und 120°C, bevorzugt zwischen 0°C und 80°C.

Bei der Durchführung des oben beschriebenen Verfahrens (b/α) zur Herstellung der Verbindungen der Formeln (IA), (IB) setzt man im Allgemeinen pro Mol acyliertem 5-Aminoisothiazol der Formel (Ia) 1 bis 2 Mol, vorzugsweise 1 bis 1,5 Mol an Halogenierungsmittel ein.

Aufarbeitung und Isolierung der Endprodukte erfolgen in allgemein bekannter Art und Weise.

Das oben beschriebene Verfahren (b/β) zur Herstellung der Verbindungen der Formeln (IA), (IB) wird mittels eines Nitrierungsreagenzes durchgeführt. Hierfür können alle üblichen Nitrierungsreagenzien eingesetzt werden. Vorzugsweise verwendbar sind Salpetersäure, gegebenenfalls in Schwefelsäure, Wasser, Essigsäure oder Acetanhydrid, N₂O₅ in Tetrachlormethan, Methylnitrat mit BF₃, Natriumnitrit in Trifluoressigsäure oder N₂O₄.

Das oben beschriebene Verfahren (b/β) zur Herstellung der Verbindungen der Formeln (IA), (IB) wird gegebenenfalls in Gegenwart eines Verdünnungsmittels durchgeführt. Vorzugsweise verwendbar sind gegebenenfalls halogenierte Kohlenwasserstoffe, wie Dichlormethan, Chloroform, Tetrachlorkohlenstoff oder Chlorbenzol oder Nitrobenzol.

Die Reaktionstemperaturen können bei dem oben beschriebenen Verfahren (b/β) in einem größeren Bereich variiert werden. Im Allgemeinen arbeitet man bei Temperaturen zwischen 0°C und 100°C, bevorzugt zwischen 0°C und 40°C.

Bei der Durchführung des oben beschriebenen Verfahrens (b/β) zur Herstellung der Verbindungen der Formeln (IA), (IB) setzt man im Allgemeinen pro Mol acyliertem 5-Aminoisothiazol der Formel (Ia) 1 bis 5 Mol, vorzugsweise 1 bis 2 Mol an Nitrierungsreagenz ein.

Aufarbeitung und Isolierung der Endprodukte erfolgen in allgemein bekannter Art und Weise.

Das oben beschriebene Verfahren (c) zur Herstellung bestimmter Verbindungen der Formeln (IA), (IB) wird mittels eines Oxidationsmittels durchgeführt.

Hierfür können übliche oxidierende Agentien eingesetzt werden. Vorzugsweise verwendbar sind Jod, Brom, Chlor oder Wasserstoffperoxid (vgl. hierzu auch J. Org. Chem. 1977, 20, 3230 und Chem. Ber. 1961, 94, 2950).

Das oben beschriebene Verfahren (c) zur Herstellung bestimmter Verbindungen der Formeln (IA), (IB) wird gegebenenfalls in Gegenwart eines Verdünnungsmittels durchgeführt. Vorzugsweise verwendbar sind gegebenenfalls halogenierte aliphatische oder aromatische Kohlenwasserstoffe, Ether, Nitrile oder Amide, wie z.B. Cyclohexan, Toluol, Chlorbenzol, Chloroform, Dichlormethan, Dichlorethan, Dioxan, Tetrahydrofuran, Diethylether, Acetonitril oder Dimethylformamid.

Die Reaktionstemperaturen können bei dem oben beschriebenen Verfahren (c) in einem größeren Bereich variiert werden. Im Allgemeinen arbeitet man bei Temperaturen zwischen -40°C und 120°C, bevorzugt zwischen 0°C und 80°C.

Aufarbeitung und Isolierung der Endprodukte erfolgen in allgemein bekannter Art und Weise.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam.

Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogodenma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Omithodoros spp., Dermanyssus gallinae, Eriophyes ribis, Phyllocoptruta oleivora, Boophilus spp., Rhipicephalus spp.; Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Die erfindungsgemäßen Verbindungen der Formeln (IA), (IB) zeichnen sich insbesondere durch eine hohe insektizide und akarizide Wirksamkeit aus.

Sie lassen sich mit besonders gutem Erfolg zur Bekämpfung von pflanzenschädigenden Insekten, wie beispielsweise gegen die Meerettichblattkäfer-Larven (Phaedon cochlaeriae), die Raupen der Kohlschabe (Plutella maculipennis), die grüne Reiszikade (Nephotettix cinctriceps) und die Raupen des Eulenfalters (Spodoptera frugiperda) oder zur Bekämpfung von pflanzenschädigenden Milben, wie beispielsweise gegen die gemeine Spinnmilbe (Tetranychus urticae) einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Naturund synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:
**Fungizide:**
   2-Aminobutan; 2-Anilino-4-methyl-6-cyclopropyl-pyrimidin; 2',6'-Dibrorno-2-methyl-4'-tnfluoromethoxy-4'-trinuoro-methyl-1,3-thiazol-5-carboxaniud; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino[alpha-(o-totyloxy)-o-tolyl]acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
   Guazatine,
   Hexachlorobenzol, Hexaconazol, Hymexazol,
   Imazalil, Imibenconazol, Iminoctadin, Iprobenfos (IBP), Iprodion, Isoprothiolan,
   Kasugamycin, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
   Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickel-dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Phthalid, Pimaricin, Piperalin,
   Polycarbamate, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram.
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
   Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin, Lambda-cyhalothrin, Lufenuron,
   Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram,
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
   Quinalphos,
   RH 5992,
   Salithion, Sebufos, Silafluofen, Sulfotep, Sulprofos,
   Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflwnuron, Trimethacarb,
   Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsfonnen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondem auch auf dem veterinärmedizinischen Sektor gegen tierische Parasiten (Ektoparasiten) wie Schildzecken, Lederzecken, Räudemilben, Laufinilben, Fliegen (stechend und leckend), parasitierende Fliegenlarven, Läuse, Haarlinge, Federlinge und Flöhe.

Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Wemeckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela gennanica, Supella spp..
Aus der Unterklasse der Acaria (Acarida) und den Ordnungen der Meta- sowie Mesostigmata z.B. Argas spp., Omithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Stemostoma spp., Varroa spp..
Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Boophilus microplus und Lucilia cuprina.

Die erfindungsgemäßen Wirkstoffe der Formeln (IA), (IB) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpudems sowie mit Hilfe von wirkstoffhaltigen Formkörpern, wie Halsbändern, Ohnnarken, Schwanzmarken, Gliedmaßenbändern, Halftern, Markierungsvorrichtungen usw.

Bei der Anwendung für Vieh, Geflügel, Haustiere etc. kann man die Wirkstoffe der Formeln (IA), (IB) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticomis, Dendrobium pertinex, Emobius mollis, Priobium carpmi, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie Sirex juvencus, Urocerus gigas, Urocerus gigas taignus, Urocerus augur.
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im Allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/ oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindelöl und/oder Monochlomaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches ÖI im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethem wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der Wo 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, genannt.

Die Herstellung und die Verwendung der erfindungsgemäßen Wirkstoffe gehen aus den nachfolgenden Beispielen hervor.

### Herstellungsbeispiele

### Beispiel 1

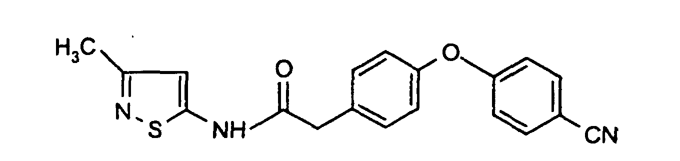

### (Verfahren a)

Zu einer Lösung von 3,01 g (0,02 Mol) 5-Amino-3-methyl-isothiazol-hydrochlorid und 3,32 g (0,042 Mol) Pyridin in 100 ml Acetonitril wird eine Lösung von 5,43 g (0,02 Mol) [4-(4-Cyano)phenoxy]-phenylessigsäurechlorid in 20 ml Acetonitril getropft. Man rührt 18 Stunden bei 25°C und engt anschließend bis zur Trockne ein. Das Reaktionsgemisch wird in Wasser/Ethylacetat aufgenommen und die organische Phase mehrmals mit 10%iger Natronlauge gewaschen. Nach Trocknen und Einengen erhält man einen zähen Rückstand, der durch Chromatographie an Kieselgel mit Ethylacetat als Laufmittel gereinigt wird.

Man erhält 2,20 g (31% der Theorie) 5-[4-(4-Cyano)phenoxy]phenylacetylamino-3-methylisothiazol.
¹HNMR (d₆-DMSO): δ = 1.18, 2.31, 3.81, 4.04, 6.74, 7.1 - 7.2, 7.40, 7.82 ppm

### Beispiel 2

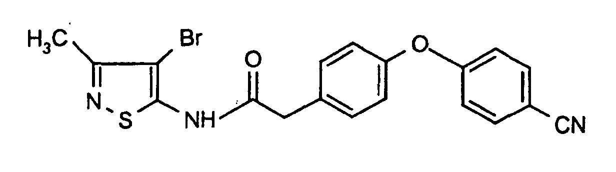

### (Verfahren b/α)

Zu einer Lösung von 0,40 g (0,00112 Mol) 5-[4-(4-Cyano)phenoxy]phenylacetylamino-3-methylisothiazol (Bsp. 1) in 10 ml Dichlormethan werden bei 5°C 0,22 g (0,00134 Mol) Brom in 1 ml Dichlormethan getropft und bei 25°C 18 Stunden gerührt. Nach Entfernen des Lösungsmittels im Vakuum erhält man 0,50 g (92 % der Theorie) 4-Brom-5-[4-cyano)phenoxy]phenylacetamino-3-methylisothiazol vom Schmelzpunkt 197 - 198°C.

Analog bzw. gemäß den allgemeinen Angaben zur Herstellung erhält man die folgenden Verbindungen der Formeln (IA), (IB):

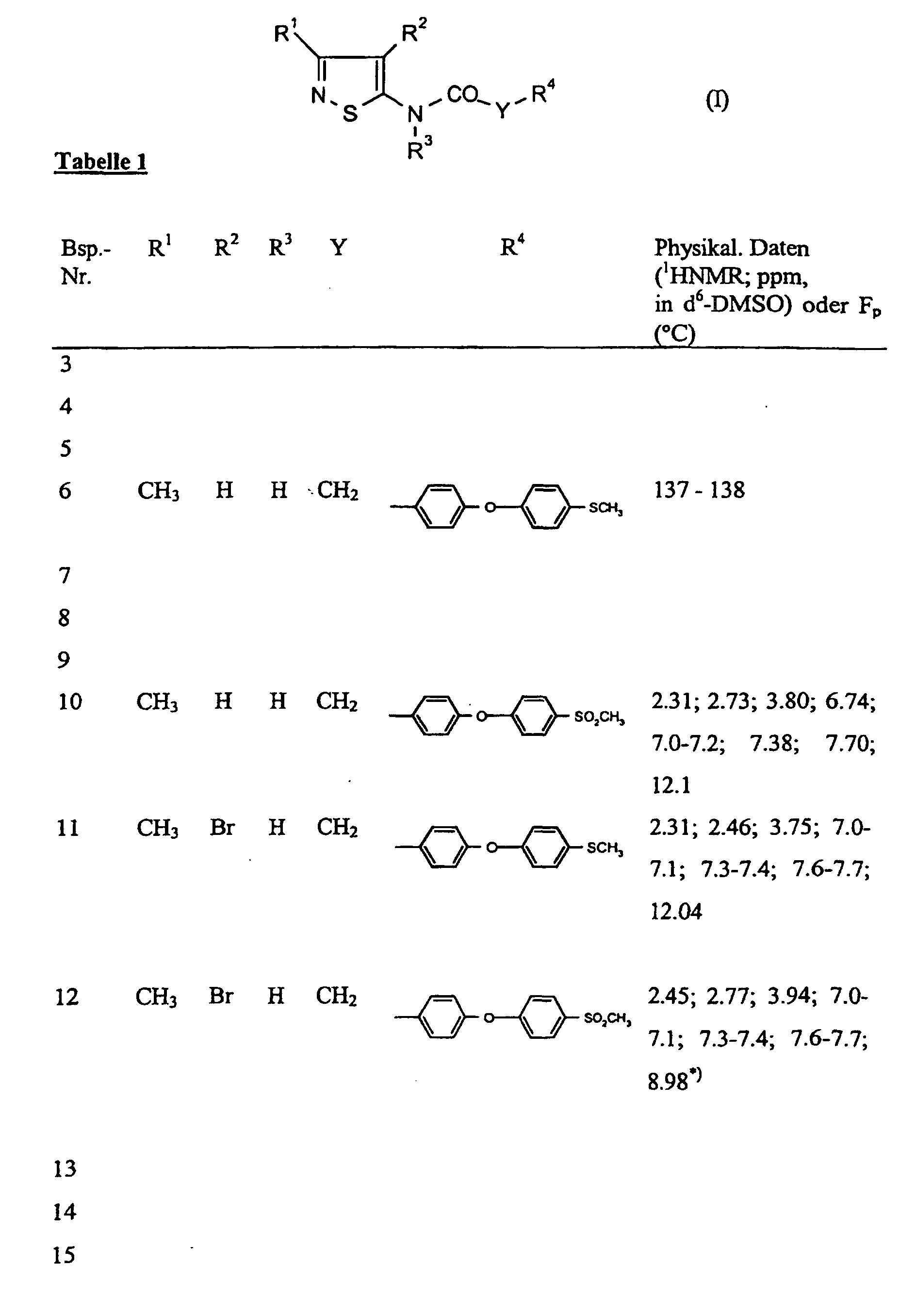

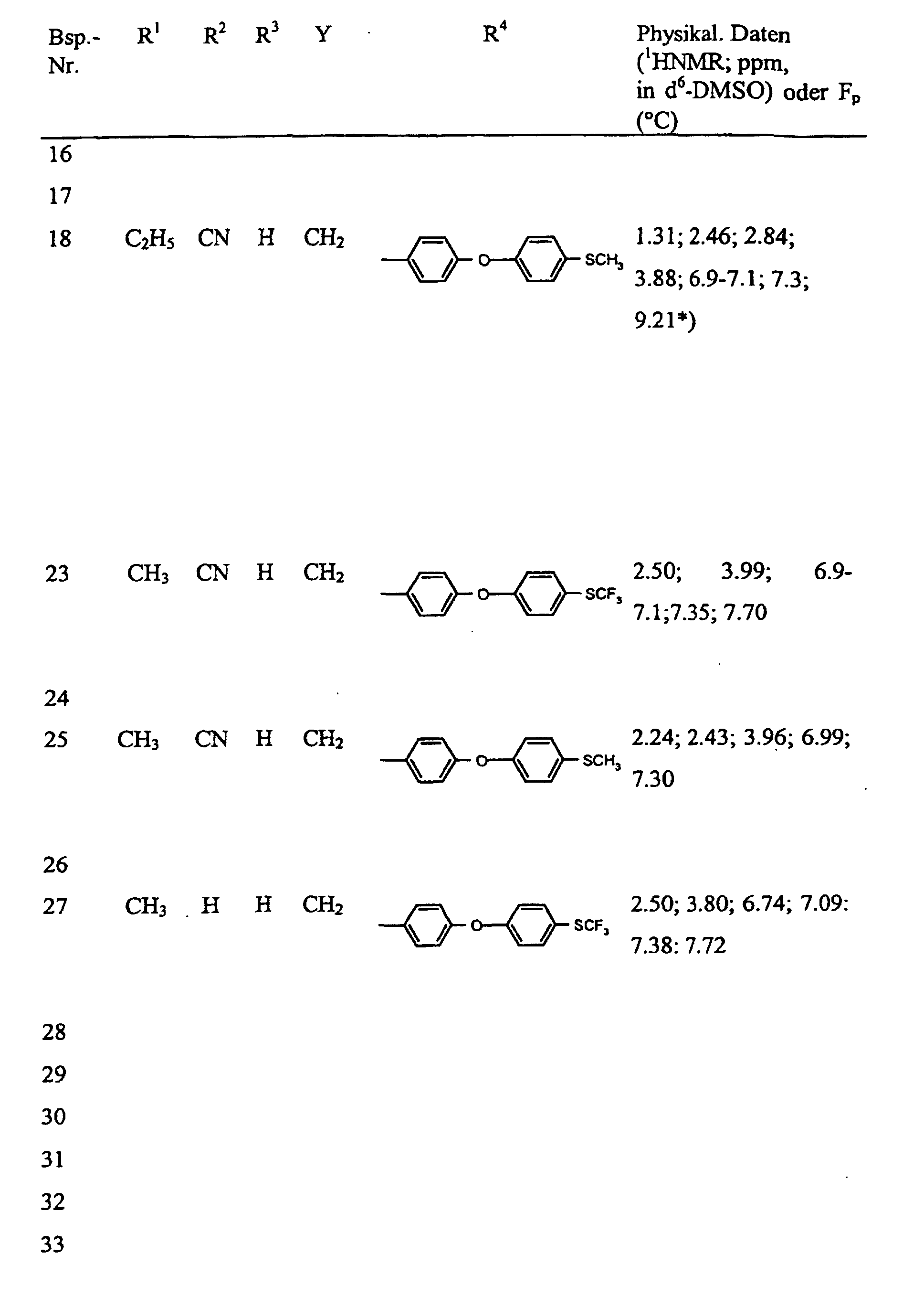

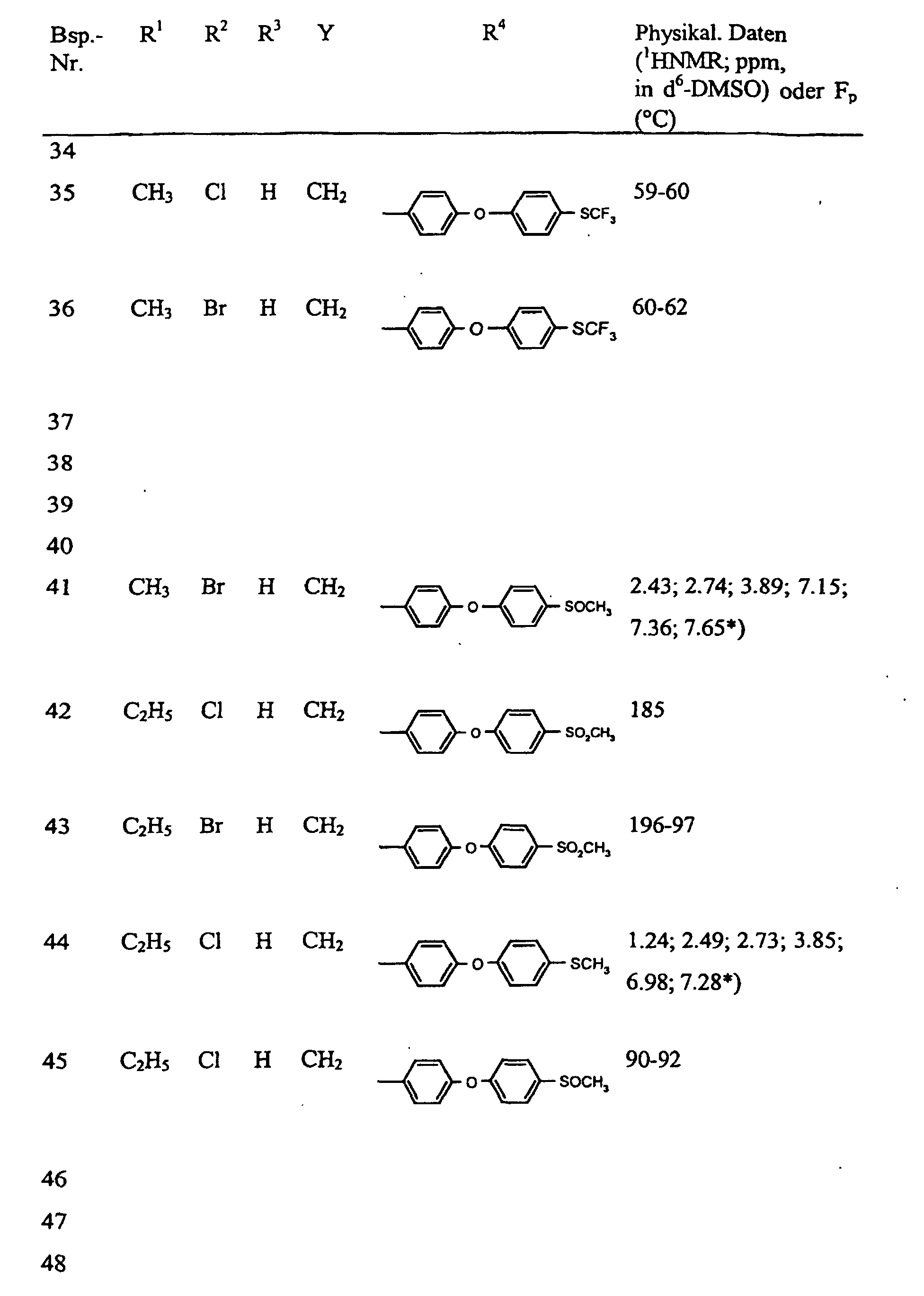

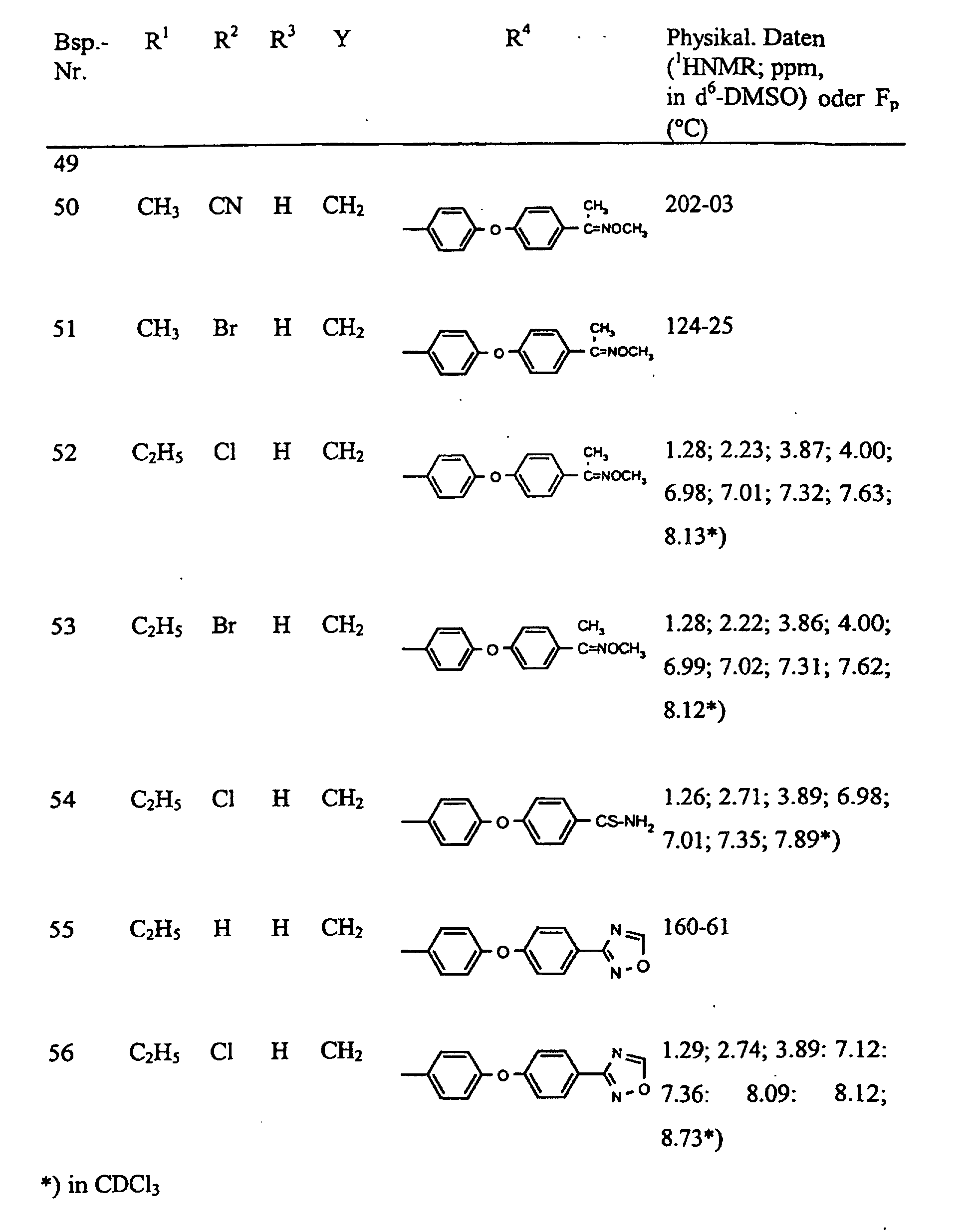

### Anwendungsbeispiele

### Beispiel A

### Phaedon-Larven-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven Phaedon cochleariae besetzt, solange die Blätter noch feucht sind.

Nach der gewünschten Zeit wird die Abtötung in % bestimmt. Dabei bedeutet 100 %, dass alle Käfer-Larven abgetötet wurden; 0 % bedeutet, dass keine Käfer-Larven abgetötet wurden.

Bei diesem Test bewirkten z.B. die Verbindungen der Herstellungsbeispiele 23, 43, 44, 50, 51, 54 und 55 bei einer beispielhaften Wirkstoffkonzentration von 0,1 % eine Abtötung von 100 % nach 7 Tagen.

### Beispiel C

### Spodoptera-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters Spodoptera frugiperda besetzt, solange die Blätter noch feucht sind.

Nach der gewünschten Zeit wird die Wirkung in % bestimmt. Dabei bedeutet 100 %, dass alle Raupen abgetötet wurden; 0 % bedeutet, dass keine Raupen abgetötet wurden.

Bei diesem Test bewirkten z.B. die Verbindungen der Herstellungsbeispiele 23, 25, 27, 35, 36, 42, 44 und 55 bei einer beispielhaften Wirkstoffkonzentration von 0,1 % eine Abtötung von 100 % nach 7 Tagen.

### Beispiel D

### Nephotettix-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade Nephotettix cincticeps besetzt, solange die Keimlinge noch feucht sind.

Nach der gewünschten Zeit wird die Abtötung in % bestimmt. Dabei bedeutet 100 %, dass alle Zikaden abgetötet wurden; 0 % bedeutet, dass keine Zikaden abgetötet wurden.

Bei diesem Test bewirkten z.B. die Verbindungen der Herstellungsbeispiele 27, 35, 41, 42 und 51 bei einer beispielhaften Wirkstoffkonzentration von 0,1 % eine Abtötung von 100 % nach 6 Tagen.

### Beispiel F

### Myzus-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test bewirkten z.B. die Verbindungen gemäß den Herstellungsbeispielen 25, 41 und 51 bei einer beispielhaften Wirkstoffkonzentration von 0,1 % eine Abtötung von mindestens 80 % nach 6 Tagen.

## Patentansprüche

1. Verbindungen der Formeln (IA) oder (IB): 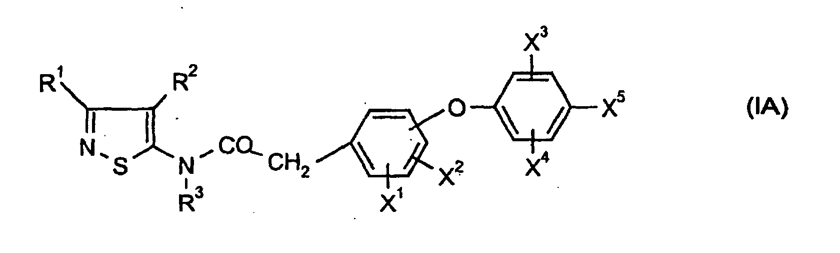 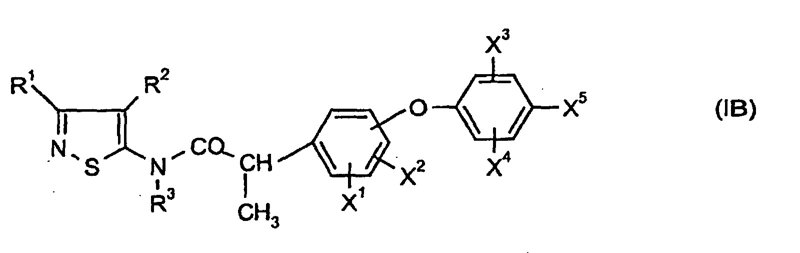 in welchen
R¹ für C₁-C₄-Alkyl, C₁-C₄-Halogenalkyl mit 1 bis 5 gleichen oder verschiedenen Halogenatomen, wie Fluor-, Chlor- und Bromatomen, C₁-C₄-Alkoxy-C₁-C₄-alkyl, C₁-C₄-Alkylthio-C₁-C₄-alkyl, C₁-C₄-Alkoxy, C₁-C₄-Alkylthio oder für gegebenenfalls einfach bis dreifach, gleich oder verschieden durch C₁-C₄-Alkyl oder Halogen substituiertes C₃-C₆-Cycloalkyl steht,
R² für Wasserstoff, Halogen, Cyano, Nitro, Thiocyanato, C₁-C₄-Alkoxycarbonyl, C₂-C₄-Alkenyloxy-carbonyl, C₁-C₄-Alkylthio, C₁-C₄-Halogenalkylthio mit 1 bis 5 gleichen oder verschiedenen Halogenatomen, wie Fluor- und Chlor, C₁-C₄-Alkylsulfinyl, C₁-C₄-Halogenalkylsulfinyl mit 1 bis 5 gleichen oder verschiedenen Halogenatomen, wie Fluor- und Chloratomen, C₁-C₄-Alkylsulfonyl, C₁-C₄-Halogenalkylsulfonyl mit 1 bis 5 gleichen oder verschiedenen Halogenatomen, wie Fluor- und Chloratomen oder für Thiocarbamoyl steht,
R³ für Wasserstoff, C₁-C₄-Alkyl, C₁-C₄-Halogenalkyl mit 1 bis 5 gleichen oder verschiedenen Halogenatomen, wie Fluor- und Chloratomen, C₁-C₄-Alkoxy-C₁-C₄-alkyl, C₁-C₄-Alkyl-carbonyl, C₁-C₄-Alkylsulfonyl, jeweils gegebenenfalls einfach bis dreifach, gleich oder verschieden im Phenylring substituiertes Phenylcarbonyl, Phenylsulfonyl oder Benzyl, wobei als Substituenten jeweils Halogen, Nitro, Cyano, C₁-C₄-Alkyl, C₁-C₂-Halogenalkyl mit 1 bis 5 gleichen oder verschiedenen Halogenatomen, wie Fluor- und Chloratome, C₁-C₄-Alkoxy, C₁-C₄-Alkylthio, C₁-C₂-Halogenalkoxy mit 1 bis 5 gleichen oder verschiedenen Halogenatomen, wie Fluor- und Choratome oder C₁-C₂-Halogenalkylthio mit 1 bis 5 gleichen oder verschiedenen Halogenatomen, wie Fluor- und Chloratome in Frage kommen, oder für gegebenenfalls einfach bis dreifach, gleich oder verschieden durch C₁-C₄-Alkyl oder Halogen substituiertes C₃-C₆-Cycloalkyl steht,
X¹, X², X³ und X⁴ für Wasserstoff, Halogen, Nitro, Cyano, C₁-C₁₂-Alkyl, C₁-C₁₂-Halogenalkyl mit 1 bis 5 gleichen oder verschiedenen Halogenatomen, wie Fluor- und Chloratome, C₁-C₁₂-Alkoxy, C₁-C₁₂-Halogenalkoxy mit 1 bis 5 gleichen oder verschiedenen Halogenatomen, wie Fluor- und Chloratome, C₁-C₁₂-Alkylthio, C₁-C₁₂-Halogenalkylthio mit 1 bis 5 gleichen oder verschiedenen Halogenatomen, wie Fluor- und Chloratomen, C₂-C₁₂-Alkenyl, C₂-C₁₂-Halogenalkenyl mit 1 bis 5 gleichen oder verschiedenen Halogenatomen, wie Fluor- und Chloratomen, gegebenenfalls einfach bis dreifach, gleich oder verschieden durch C₁-C₄-Alkyl oder Halogen substituiertes C₃-C₈-Cycloalkyl, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen Halogenatomen, wie Fluor- und Chloratomen, C₁-C₄-Alkyl-carbonyl, C₁-C₄-Alkoxy-carbonyl, Thioamid, C₁-C₄-Alkoximino-C₁-C₄-alkyl oder gegebenenfalls durch C₁-C₄-Alkyl substituiertes Oxadiazolyl stehen, C₁-C₄-Alkylthio, C₁-C₄-Halogenalkylthio mit 1 bis 5 gleichen oder verschiedenen Halogenatomen, wie Fluor- und Chloratome, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen Halogenatomen, wie Fluor- und Chloratomen, C₁-C₄-Alkoxy-carbonyl, Thioamid, C₁-C₄-Alkoximino-C₁-C₄-alkyl oder gegebenenfalls durch C₁-C₄-Alkyl substituiertes Oxdiazolyl stehen,
X⁵ für Halogen, Nitro, Cyano, C₁-C₁₂-Alkyl, C₁-C₁₂-Halogenalkyl mit 1 bis 5 gleichen oder verschiedenen Halogenatomen, wie Fluor- und Chloratome, C₁-C₁₂-Alkoxy, C₁-C₁₂-Halogenalkoxy mit 1 bis 5 gleichen oder verschiedenen Halogenatomen, wie Fluor- und Chloratome, C₁-C₁₂-Alkylthio, C₁-C₁₂-Halogenalkylthio mit 1 bis 5 gleichen oder verschiedenen Halogenatomen, wie Fluor- und Chloratomen, C₂-C₁₂-Alkenyl, C₂-C₁₂-Halogenalkenyl mit 1 bis 5 gleichen oder verschiedenen Halogenatomen, wie Fluor- und Chloratomen, gegebenenfalls einfach bis dreifach, gleich oder verschieden durch C₁-C₄-Alkyl oder Halogen substituiertes C₃-C₈-Cycloalkyl, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen Halogenatomen, wie Fluor- und Chloratomen, C₁-C₄-Alkyl-carbonyl, C₁-C₄-Alkoxy-carbonyl, Thioamid, C₁-C₄-Alkoximino-C₁-C₄-alkyl oder gegebenenfalls durch C₁-C₄-Alkyl substituiertes Oxadiazolyl stehen, C₁-C₄-Alkylthio, C₁-C₄-Halogenalkylthio mit 1 bis 5 gleichen oder verschiedenen Halogenatomen, wie Fluor- und Chloratome, C₁-C₄-Alkylthio-C₁-C₄-alkyl, C₁-C₄-Alkylsulfonyl, C₁-C₄-Halogenalkylsulfonyl mit 1 bis 5 gleichen oder verschiedenen Halogenatomen, wie Fluor- und Chloratomen, C₁-C₄-Alkoxy-carbonyl, Thioamid, C₁-C₄-Alkoximino-C₁-C₄-alkyl oder gegebenenfalls durch C₁-C₄-Alkyl substituiertes Oxdiazolyl stehen.

2. Verbindungen der Formeln (IA) oder (IB) gemäß Anspruch 1, in welchen der Phenoxyrest in para-Stellung zur -NR³-CO-CH₂- bzw. -NR³-CO-CH(CH₃)-Gruppe steht.

3. Verbindungen gemäß einem der Ansprüche 1 oder 2, in welchen die Substituenten X¹, X², X³ und X⁴ für Wasserstoff stehen.

4. Verfahren zur Herstellung von Verbindungen der Formel (I) gemäß Anspruch 1, **dadurch gekennzeichnet, dass** man
a) 5-Aminoisothiazole der Formel (II) 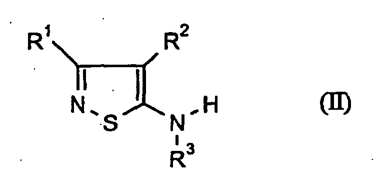 in welcher
R¹, R² und R³ die in Anspruch 1 angegebene Bedeutung haben,
mit Säurehalogeniden der Formel (III)
R⁴-Y-CO-Hal (III)
in welcher
R⁴-Y für den Rest 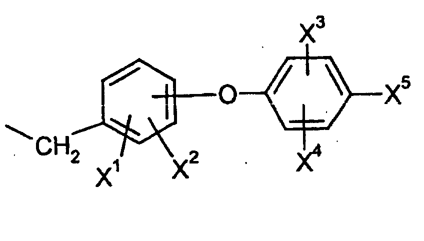 oder für den Rest 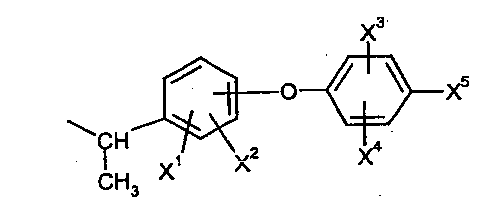 steht und X¹, X², X³, X⁴ und X⁵ die in einem der vorstehenden Ansprüche angegebenen Bedeutungen haben,
Hal für Halogen steht,
in Gegenwart einer Base und in Gegenwart eines Verdünnungsmittels umsetzt; oder
b) acylierte 5-Aminoisothiazole der Formel (Ia) 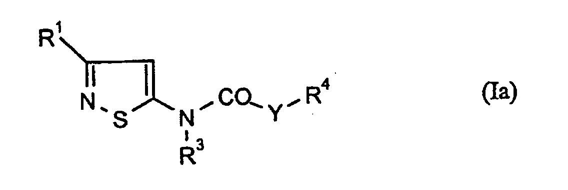 in welcher
R¹ und R³ die in den vorstehenden Ansprüchen angegebene Bedeutung haben, und
R⁴-Y- die unter a) angegebene Bedeutung hat,
(α) mit einem Halogenierungsmittel, gegebenenfalls in Gegenwart eines Verdünnungsmittels sowie gegebenenfalls in Gegenwart eines Katalysators umsetzt,
oder
(β) mit einem Nitrierungsreagenz, gegebenenfalls in Gegenwart eines Verdünnungsmittels umsetzt,
oder
c) β-Amino-thiocrotonamide der Formel (IV) 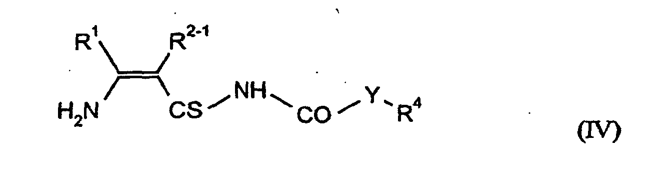 in welcher
R¹ die in den vorstehenden Ansprüchen angegebene Bedeutung hat,
R⁴-Y- die unter a) angegebene Bedeutung hat und
R²⁻¹ für Cyano oder Alkoxycarbonyl steht,
mit einem Oxidationsmittel, gegebenenfalls in Gegenwart eines Verdünnungsmittels cyclisiert.

5. Verbindungen der Formel (IV) 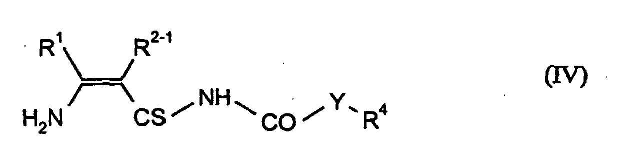 in welcher
R¹ die in Anspruch 1 angegebene Bedeutung hat
R⁴-Y- die in Anspruch 4 angegebene Bedeutung hat und
R²⁻¹ für Cyano oder Alkoxycarbonyl steht.

6. Schädlingsbekämpfungsmittel, **gekennzeichnet durch** einen Gehalt an mindestens einer Verbindung der Formel (I) gemäß Anspruch 1.

7. Verwendung von Verbindungen der Formel (I) gemäß Anspruch 1 zur Bekämpfung von Schädlingen.

8. Verfahren zur Bekämpfung von Schädlingen, **dadurch gekennzeichnet, dass** man Verbindungen der Formel (I) gemäß Anspruch 1 auf Schädlinge und/oder ihren Lebensraum einwirken lässt.

9. Verfahren zur Herstellung von Schädlingsbekämpfungsmitteln, **dadurch gekennzeichnet, dass** man Verbindungen der Formel (I) gemäß Anspruch 1 mit Streckmitteln und/oder oberflächenaktiven Mitteln vermischt.

10. Verwendung von Verbindungen der Formel (I) gemäß Anspruch 1 zur Herstellung von Schädlingsbekämpfungsmitteln.

## Claims

1. Compounds of the formulae (IA) or (IB) 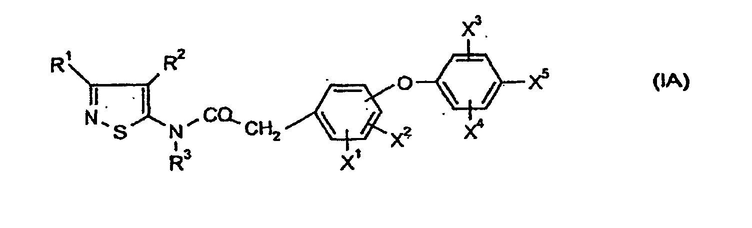 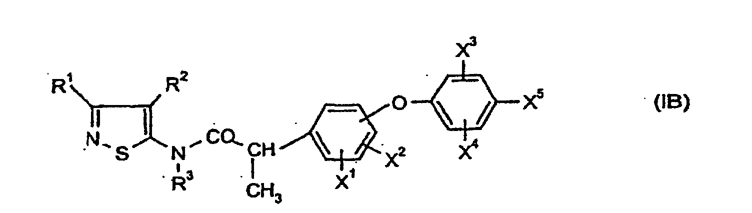 in which
R¹ represents C₁-C₄-alkyl, C₁-C₄-halogenoalkyl having 1 to 5 identical or different halogen atoms, such as fluorine, chlorine and bromine atoms, C₁-C₄-alkoxy-C₁-C₄-alkyl, C₁-C₄-alkylthio-C₁-C₄-alkyl, C₁-C₄-alkoxy or C₁-C₄-alkylthio, or represents C₃-C₆-cycloalkyl which is optionally substituted once to three times in an identical or different manner by C₁-C₄-alkyl or halogen,
R² represents hydrogen, halogen, cyano, nitro, thiocyanato, C₁-C₄-alkoxy-carbonyl, C₂-C₄-alkenyloxy-carbonyl, C₁-C₄-alkylthio, C₁-C₄-halogenoalkylthio having 1 to 5 identical or different halogen atoms, such as fluorine and chlorine atoms, C₁-C₄-alkylsulphinyl, C₁-C₄-halogenoalkylsulphinyl having 1 to 5 identical or different halogen atoms, such as fluorine and chlorine atoms, C₁-C₄-alkylsulphonyl or C₁-C₄-halogenoalkylsulphonyl having 1 to 5 identical or different halogen atoms, such as fluorine and chlorine atoms, or represents thiocarbamoyl,
R³ represents hydrogen, C₁-C₄-alkyl, C₁-C₄-halogenoalkyl having 1 to 5 identical or different halogen atoms, such as fluorine and chlorine atoms, C₁-C₄-alkoxy-C₁-C₄-alkyl, C₁-C₄-alkyl-carbonyl, C₁-C₄-alkyl-sulphonyl or phenylcarbonyl, phenylsulphonyl or benzyl, in each case optionally substituted in the phenyl ring once to three times in an identical or different manner, possible substituents in each case being halogen, nitro, cyano, C₁-C₄-alkyl, C₁-C₂-halogenoalkyl having 1 to 5 identical or different halogen atoms, such as fluorine and chlorine atoms, C₁-C₄-alkoxy, C₁-C₄-alkylthio, C₁-C₂-halogenoalkoxy having 1 to 5 identical or different halogen atoms, such as fluorine and chlorine atoms, or C₁-C₂-halogenoalkylthio having 1 to 5 identical or different halogen atoms, such as fluorine and chlorine atoms, or represents C₃-C₆-cycloalkyl which is optionally substituted once to three times in an identical or different manner by C₁-C₄-alkyl or halogen,
X¹, X², X³ and X⁴ represent hydrogen, halogen, nitro, cyano, C₁-C₁₂-alkyl, C₁-C₁₂-halogenoalkyl having 1 to 5 identical or different halogen atoms, such as fluorine and chlorine atoms, C₁-C₁₂-alkoxy, C₁-C₁₂-halogenoalkoxy having 1 to 5 identical or different halogen atoms, such as fluorine and chlorine atoms, C₁-C₁₂-alkylthio, C₁-C₁₂-halogenoalkylthio having 1 to 5 identical or different halogen atoms, such as fluorine and chlorine atoms, C₂-C₁₂-alkenyl, C₂-C₁₂-halogenoalkenyl having 1 to 5 identical or different halogen atoms, such as fluorine and chlorine atoms, C₃-C₈-cycloalkyl which is optionally substituted once to three times in an identical or different manner by C₁-C₄-alkyl or halogen, C₁-C₄-alkylthio-C₁-C₄-alkyl, C₁-C₄-alkylsulphonyl C₁-C₄-halogenoalkylsulphonyl having 1 to 5 identical or different halogen atoms, such as fluorine and chlorine atoms, C₁-C₄-alkyl-carbonyl, C₁-C₄-alkyl-alkoxy-carbonyl, thioamide, C₁-C₄-alkoximino-C₁-C₄-alkyl or optionally C₁-C₄-alkyl-substituted oxadiazolyl, or represent C₁-C₄-alkylthio, C₁-C₄-halogenoalkoxythio having 1 to 5 identical or different halogen atoms, such as fluorine and chlorine atoms, C₁-C₄-alkylthio-C₁-C₄-alkyl, C₁-C₄-alkylsulphonyl, C₁-C₄-halogenoalkylsulphonyl having 1 to 5 identical or different halogen atoms, such as fluorine or chlorine atoms, C₁-C₄-alkoxy-carbonyl, thioamide, C₁-C₄-alkoximino-C₁-C₄-alkyl or optionally C₁-C₄-alkyl-substituted oxdiazolyl and
X⁵ represents halogen, nitro, cyano, C₁-C₁₂-alkyl, C₁-C₁₂-halogenoalkyl having 1 to 5 identical or different halogen atoms, such as fluorine and chlorine atoms, C₁-C₁₂-alkoxy, C₁-C₁₂-halogenoalkoxy having 1 to 5 identical or different halogen atoms, such as fluorine and chlorine atoms, C₁-C₁₂-alkylthio, C₁-C₁₂-halogenoalkylthio having 1 to 5 identical or different halogen atoms, such as fluorine and chlorine atoms, C₂-C₁₂-alkenyl, C₂-C₁₂-halogenoalkenyl having 1 to 5 identical or different halogen atoms, such as fluorine and chlorine atoms, optionally singly to triply, identically or differently C₁-C₄-alkyl- or halogen-substituted C₃-C₈-cycloalkyl, C₁-C₄-alkylthio-C₁-C₄-alkyl, C₁-C₄-alkylsulphonyl, C₁-C₄-halogenoalkyl-sulphonyl having 1 to 5 identical or different halogen atoms, such as fluorine and chlorine atoms, C₁-C₄-alkyl-carbonyl, C₁-C₄-alkoxy-carbonyl, thioamide, C₁-C₄-alkoxyimino-C₁-C₄-alkyl or optionally C₁-C₄-alkyl-substituted oxadiazolyl, C₁-C₄-alkylthio, C₁-C₄-halogenoalkylthio having 1 to 5 identical or different halogen atoms, such as fluorine and chlorine atoms, C₁-C₄-alkylthio-C₁-C₄-alkyl, C₁-C₄-alkylsulphonyl, C₁-C₄-halogenoalkyl-sulphonyl having 1 to 5 identical or different halogen atoms, such as fluorine and chlorine atoms, C₁-C₄-alkoxy-carbonyl, thioamide, C₁-C₄-alkoximino-C₁-C₄-alkyl or optionally C₁-C₄-alkyl-substituted oxdiazolyl.

2. Compounds of the formulae (IA) or (IB) as per Claim 1, in which the phenoxy radical is disposed para to the -NR³-CO-CH₂ or -NR³-CO-CH(CH₃) group.

3. Compounds as per one of Claims 1 or 2, in which the substituents X¹, X², X³ and X⁴ represent hydrogen.

4. Process for the preparation of compounds of the formula (I) according to Claim 1, **characterized in that**
a) 5-aminoisothiazoles of the formula (II) 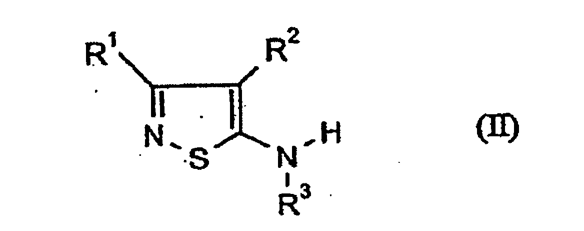 in which
R¹, R² and R³ have the meaning given in Claim 1,
are reacted with acid halides of the formula (III)
R⁴-Y-CO-Hal (III)
in which
R⁴-Y represents the radical 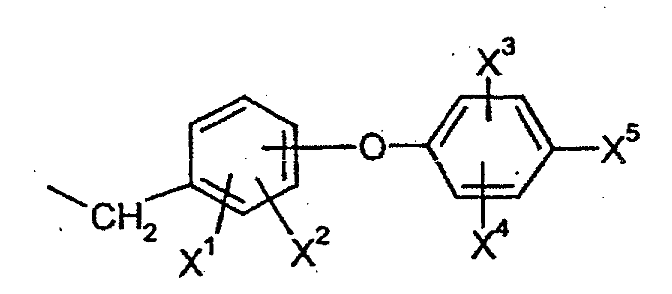 or represents the radical 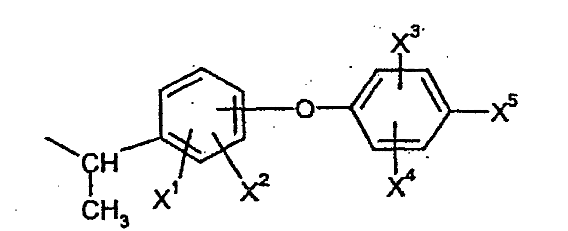 and X¹, X², X³, X⁴ and X⁵ have the meanings given in one of the preceding claims, and
Hal represents halogen,
in the presence of a base and in the presence of a diluent;
or
b) acylated 5-aminoisothiazoles of the formula (Ia) 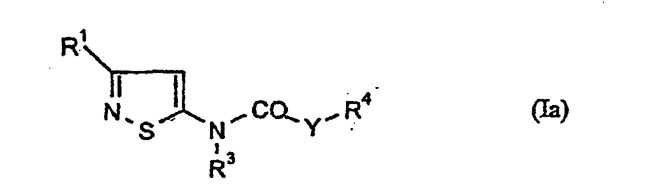 in which
R¹ and R³ have the meaning given in the preceding claims, and
R⁴-Y- has the meaning given under a),
(α) are reacted with a halogenating agent, if appropriate in the presence of a diluent and if appropriate in the presence of a catalyst,
or
(β) are reacted with a nitrating reagent, if appropriate in the presence of a diluent,
or
c) β-amino-thiocrotonamides of the formula (IV) 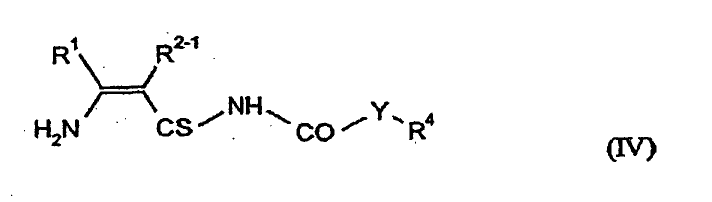 in which
R¹ has the meaning given in the preceding claims,
R⁴-Y- has the meaning given under a), and
R²⁻¹ represents cyano or alkoxycarbonyl,
are cyclized with an oxidizing agent, if appropriate in the presence of a diluent.

5. Compounds of the formula (IV) 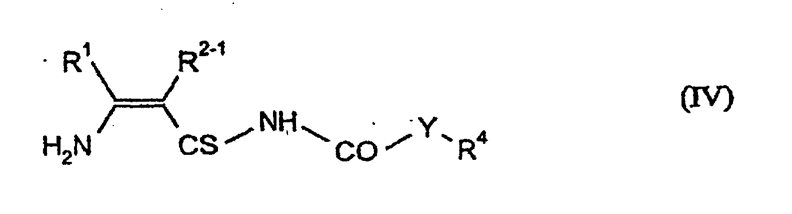 in which
R¹ has the meaning given in Claim 1,
R⁴-Y- has the meaning given in Claim 4, and
R²⁻¹ represents cyano or alkoxycarbonyl.

6. Compositions for controlling pests, **characterized in that** they contain at least one compound of the formula (I) according to Claim 1.

7. Use of compounds of the formula (I) according to Claim 1 for controlling pests.

8. Method of controlling pests, **characterized in that** compounds of the formula (I) according to Claim 1 are allowed to act on pests and/or their environment.

9. Process for the preparation of compositions for controlling pests, **characterized in that** compounds of the formula (I) according to Claim 1 are mixed with extenders and/or surface-active agents.

10. Use of compounds of the formula (I) according to Claim 1 for the preparation of compositions for controlling pests.

## Revendications

1. Composés de formules (IA) ou (IB) : 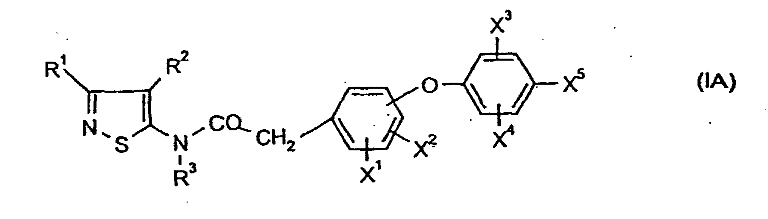 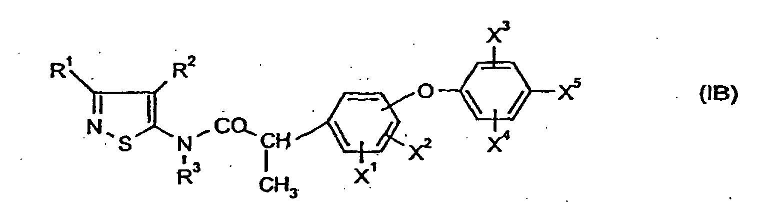 dans lesquelles
R¹ représente un reste alkyle en C₁ à C₄, halogénalkyle en C₁ à C₄ ayant 1 à 5 atomes d'halogène identiques ou différents, tels que des atomes de fluor, chlore et brome, (alkoxy en C₁ à C₄)-(alkyle en C₁ à C₄), (alkylthio en C₁ à C₄)-(alkyle en C₁ à C₄), alkoxy en C₁ à C₄, alkylthio en C₁ à C₄ ou un reste cycloalkyle en C₃ à C₆ éventuellement substitué 1 à 3 fois identiques ou différentes par un radical alkyle en C₁ à C₄ ou halogéno,
R² représente l'hydrogène, un halogène, un groupe cyano, nitro, thiocyanato, un reste (alkoxy en C₁ à C₄)-(carbonyle), (alcényloxy en C₂ à C₄)-carbonyle, alkylthio en C₁ à C₄, halogénalkylthio en C₁ à C₄ ayant 1 à 5 atomes d'halogène identiques ou différents tels que des atomes de fluor et de chlore, alkylsulfinyle en C₁ à C₄, halogénalkyl-sulfinyle en C₁ à C₄ ayant 1 à 5 atomes d'halogène identiques ou différents, tels que des atomes de fluor et de chlore, alkylsulfonyle en C₁ à C₄, halogénalkyl-sulfonyle en C₁ à C₄ ayant 1 à 5 atomes d'halogène identiques ou différents, tels que des atomes de fluor et de chlore, ou un reste thiocarbamoyle,
R³ représente l'hydrogène, un reste alkyle en C₁ à C₄, halogénalkyle en C₁ à C₄ ayant 1 à 5 atomes d'halogène identiques ou différents, tels que des atomes de fluor et de chlore, (alkoxy en C₁ à C₄)-(alkyle en C₁ à C₄), (alkyle en C₁ à C₄)carbonyle, alkyl-sulfonyle en C₁ à C₄, un reste phénylcarbonyle, phénylsulfonyle ou benzyle dont chacun est éventuellement substitué 1 à 3 fois identiques ou différentes dans le noyau phényle, en considérant dans chaque cas comme substituants un radical halogéno, nitro, cyano, alkyle en C₁ à C₄, halogénalkyle en C₁ ou C₂ ayant 1 à 5 atomes d'halogène identiques ou différents tels que des atomes de fluor et de chlore, un radical alkoxy en C₁ à C₄, alkylthio en C₁ à C₄, halogénalkoxy en C₁ ou C₂ ayant 1 à 5 atomes d'halogène identiques ou différents tels que des atomes de fluor et de chlore ou un radical halogénalkylthio en C₁ ou C₂ ayant 1 à 5 atomes d'halogène identiques ou différents tels que des atomes de fluor et de chlore, ou bien un reste cycloalkyle en C₃ à C₆ portant éventuellement 1 à 3 substituants, identiques ou différents, alkyle en C₁ à C₄ ou halogéno,
X¹ ,X², X³ et X⁴ représentent l'hydrogène, un halogène, un groupe nitro, cyano, un reste alkyle en C₁ à C₁₂, halogénalkyle en C₁ à C₁₂ ayant 1 à 5 atomes d'halogène identiques ou différents tels que des atomes de fluor et de chlore, alkoxy en C₁ à C₁₂, halogénalkoxy en C₁ à C₁₂ ayant 1 à 5 atomes d'halogène identiques ou différents, tels que des atomes de fluor et de chlore, alkylthio en C₁ à C₁₂, halogénalkylthio en C₁ à C₁₂ ayant 1 à 5 atomes d'halogène identiques ou différents, tels que des atomes de fluor et de chlore, alcényle en C₂ à C₁₂, halogénalcényle en C₂ à C₁₂ ayant 1 à 5 atomes d'halogène identiques ou différents, tels que des atomes de fluor et de chlore, un reste cycloalkyle en C₃ à C₈ éventuellement substitué 1 à 3 fois identiques ou différentes par un radical alkyle en C₁ à C₄ ou un halogène, un reste (alkylthio en C₁ à C₄)-(alkyle en C₁ à C₄), alkyl-sulfonyle en C₁ à C₄, halogénalkyl-sulfonyle en C₁ à C₄ ayant 1 à 5 atomes d'halogène identiques ou différents, tels que des atomes de fluor et de chlore, (alkyle en C₁ à C₄)carbonyle, (alkoxy en C₁ à C₄)carbonyle, thio-amide, (alkoximino en C₁ à C₄)(alkyle en C₁ à C₄) ou un reste oxadiazolyle éventuellement substitué par un radical alkyle en C₁ à C₄, un reste alkylthio en C₁ à C₄, halogénalkylthio en C₁ à C₄ ayant 1 à 5 atomes d'halogène identiques ou différents, tels que des atomes de fluor et de chlore, (alkylthio en C₁ à C₄)-(alkyle en C₁ à C₄), alkylsulfonyle en C₁ à C₄, halogénalkyl-sulfonyle en C₁ à C₄ ayant 1 à 5 atomes d'halogène identiques ou différents, tels que des atomes de fluor et de chlore, (alkoxy en C₁ à C₄)carbonyle, thio-amide, (alkoximino en C₁ à C₄)-(alkyle en C₁ à C₄) ou oxdiazolyle éventuellement substitué par un radical alkyle en C₁ à C₄,
X⁵ représente un halogène, un groupe nitro, cyano, un reste alkyle en C₁ à C₁₂, halogénalkyle en C₁ à C₁₂ ayant 1 à 5 atomes d'halogène identiques ou différents, tels que des atomes de fluor et de chlore, un reste alkoxy en C₁ à C₁₂, halogénalkoxy en C₁ à C₁₂ ayant 1 à 5 atomes d'halogène identiques ou différents, tels que des atomes de fluor et de chlore, un reste alkylthio en C₁ à C₁₂, halogénalkylthio en C₁ à C₁₂ ayant 1 à 5 atomes d'halogène identiques ou différents, tels que des atomes de fluor et de chlore, un reste alcényle en C₂ à C₁₂, halogénalcényle en C₂ à C₁₂ ayant 1 à 5 atomes d'halogène identiques ou différents, tels que des atomes de fluor et de chlore, un reste cycloalkyle en C₃ à C₈ éventuellement substitué 1 à 3 fois identiques ou différentes par un radical alkyle en C₁ à C₄ ou halogéno, un reste (alkylthio en C₁ à C₄)-(alkyle en C₁ à C₄), alkylsulfonyle en C₁ à C₄, halogénalkyl-sulfonyle en C₁ à C₄ ayant 1 à 5 atomes d'halogène identiques ou différents, tels que des atomes de fluor et de chlore, un reste (alkyle en C₁ à C₄) carbonyle, (alkoxy en C₁ à C₄)carbonyle, thio-amide, (alkoximino en C₁ à C₄)-(alkyle en C₁ à C₄) ou un reste oxadiazolyle éventuellement substitué par un radical alkyle en C₁ à C₄, un reste alkylthio en C₁ à C₄, halogénalkylthio en C₁ à C₄ ayant 1 à 5 atomes d'halogène identiques ou différents, tels que des atomes de fluor et de chlore, un reste (alkylthio en C₁ à C₄) - (alkyle en C₁ à C₄), alkylsulfonyle en C₁ à C₄, halogénalkyl-sulfonyle en C₁ à C₄ ayant 1 à 5 atomes d'halogène identiques ou différents, tels que des atomes de fluor et de chlore, un reste (alkoxy en C₁ à C₄)carbonyle, thio-amide, (alkoximino en C₁ à C₄) - (alkyle en C₁ à C₄) ou un reste oxdiazolyle éventuellement substitué par un radical alkyle en C₁ à C₄.

2. Composés de formules (IA) ou (IB) suivant la revendication 1, dans lesquelles le reste phénoxy est en position para- par rapport au groupe -NR³-CO-CH₂- ou respectivement -NR³-CO-CH(CH₃)-.

3. Composés suivant la revendication 1 ou 2, dans lesquels les substituants X¹, X², X³ et X⁴ représentent l'hydrogène.

4. Procédé de production de composés de formule (I) suivant la revendication 1, **caractérisé en ce que** :
a) on fait réagir des 5-amino-isothiazole de formule (II) 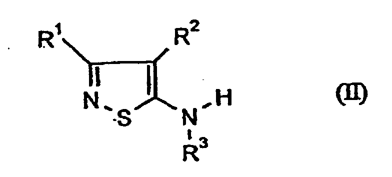 dans laquelle
R¹, R² et R³ ont la définition indiquée dans la revendication 1,
avec des halogénures d'acides de formule (III)
R⁴-Y-CO-Hal (III)
dans laquelle
R⁴-Y représente le reste 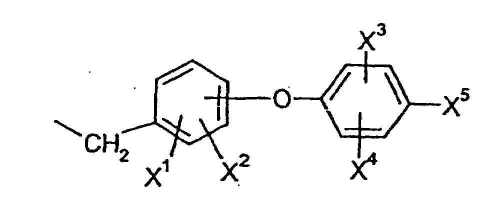 ou le reste 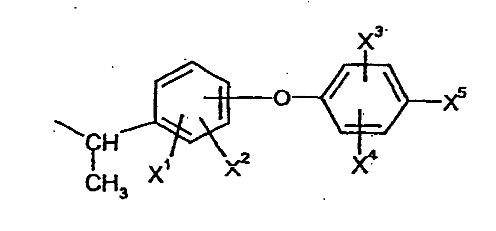
et X¹, X², X³, X⁴ et X⁵ ont les définitions indiquées dans l'une des revendications précédentes,
Hal représente un halogène,
en présence d'une base et en présence d'un diluant ;
ou bien
b) on fait réagir des 5-amino-isothiazole acylés de formule (Ia) 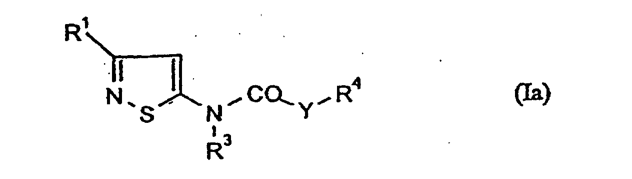 dans laquelle
R¹ et R³ ont la définition indiquée dans les revendications précédentes, et
R⁴-Y a la définition indiquée en a),
(α) avec un agent d'halogénation, éventuellement en présence d'un diluant ainsi que le cas échéant en présence d'un catalyseur,
ou bien
(β) avec un réactif de nitration, éventuellement en présence d'un diluant,
ou bien
c) on cyclise des β-aminothiocrotonamide de formule (IV) 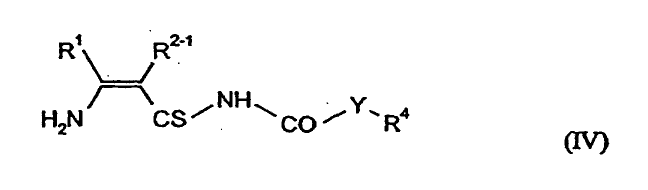 dans laquelle
R¹ a la définition indiquée dans les revendications précédentes,
R⁴-Y- a la définition indiquée en a) et,
R²⁻¹ est un groupe cyano ou alkoxycarbonyle, avec un agent oxydant, éventuellement en présence d'un diluant.

5. Composés de formule (IV) 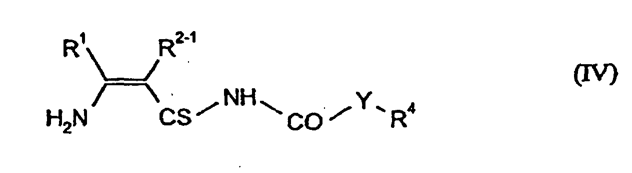 dans laquelle
R¹ a la définition indiquée dans la revendication 1,
R⁴-Y- a la définition indiquée dans la revendication 4, et
R²⁻¹ est un groupe cyano ou alkoxycarbonyle.

6. Composition pesticide, **caractérisée par** une teneur en au moins un composé de formule (I) suivant la revendication 1.

7. Utilisation de composés de formule (I) suivant la revendication 1 pour combattre des parasites.

8. Procédé de lutte contre des parasites, **caractérisé en ce qu'**on fait agir des composés de formule (I) suivant la revendication 1 sur les parasites et/ou sur leur milieu.

9. Procédé de préparation de compositions pesticides, **caractérisé en ce qu'**on mélange des composés de formule (I) suivant la revendication 1 avec des diluants et/ou avec des agents tensio-actifs.

10. Utilisation de composés de formule (I) suivant la revendication 1 pour la préparation de compositions pesticides.
